# EUROPÄISCHE PATENTANMELDUNG

(11) **EP 2 664 329 A1**
(43) Veröffentlichungstag der Anmeldung: **20.11.2013**
(21) Anmeldenummer: 12168139.9
(22) Anmeldetag: 15.05.2012
(51) Int. Cl.: A61K 31/202, A61K 9/00, A61K 9/06, A61K 9/107, A61K 33/00, A61P 27/02

(54) **Ophthalmologisches Vehikelsystem**

(71) Anmelder: F. Holzer GmbH, 66386 St. Ingbert (DE)
(72) Erfinder: Steinfeld, Ute, 66386 St. Ingbert (DE); Holzer, Frank, 66386 St. Ingbert (DE); Lee, Hyeck Hee, 66386 St. Ingbert (DE); Mahler, Markus, 66333 Völklingen (DE)
(74) Vertreter: Pfenning, Meinig & Partner GbR

(57) **Zusammenfassung**

Die vorliegende Erfindung betrifft ein ophthalmologisches Vehikelsystem zur Permeation und/oder zum Wirkstofftransport von ophthalmologischen Wirkstoffen durch die Hornhaut (Cornea) und/oder die Bindehaut (Sklera) des Auges eines Säugetieres. Dieses Vehikelsystem begünstigt den Transport der Wirkstoffe durch die Hornhaut und/oder das Bindegewebe des Auges. Das Vehikelsystem eignet sich zur Prophylaxe und/oder Behandlung von Erkrankungen des vorderen, und/oder hinteren Augenabschnittes. Ebenso betrifft die vorliegende Erfindung ein ophthalmologisches Kit, umfassend eine spezielle ophthalmologische Zusammensetzung sowie als separate Formulierung einen ophthalmologischen Wirkstoff. Zudem ist die Verwendung einer speziellen ophthalmologischen Zusammensetzung als Vehikelsystem, Penetrationsbeschleuniger, Penetrationsenhancer, Absorptionsenhancer/-verbesserer/- beschleuiger zur Permeation und/oder zum Wirkstofftranport von ophthalmologischen Wirkstoffen durch die Hornhaut (Cornea) und/oder die Bindehaut (Sklera) des Auges eines Säugetieres Gegenstand der Erfindung. Weiterhin betrifft die Erfindung einen Fluidspender, der ein erfindungsgemäßes ophthalmologisches Vehikelsystem beinhaltet.

## Beschreibung

Die vorliegende Erfindung betrifft ein ophthalmologisches Vehikelsystem zur Permeation und/oder zum Wirkstofftransport von ophthalmologischen Wirkstoffen durch die Hornhaut (Cornea) und/oder die Bindehaut (Sklera) des Auges eines Säugetieres. Dieses Vehikelsystem begünstigt den Transport der Wirkstoffe durch die Hornhaut und/oder das Bindegewebe des Auges. Das Vehikelsystem eignet sich zur Prophylaxe und/oder Behandlung von Erkrankungen des vorderen, und/oder hinteren Augenabschnittes. Ebenso betrifft die vorliegende Erfindung ein ophthalmologisches Kit, umfassend eine spezielle ophthalmologische Zusammensetzung sowie als separate Formulierung einen ophthalmologischen Wirkstoff. Zudem ist die Verwendung einer speziellen ophthalmologischen Zusammensetzung als Vehikelsystem, Penetrationsbeschleuniger, Penetrationsenhancer, Absorptionsenhancer/-verbesserer/- beschleuiger zur Permeation und/oder zum Wirkstofftranport von ophthalmologischen Wirkstoffen durch die Hornhaut (Cornea) und/oder die Bindehaut (Sklera) des Auges eines Säugetieres Gegenstand der Erfindung. Weiterhin betrifft die Erfindung einen Fluidspender, der ein erfindungsgemäßes ophthalmologisches Vehikelsystem beinhaltet.

Topisch auf die Cornea applizierte Wirkstoffe erreichen oft nicht oder nicht in therapeutischen Konzentrationen das Augeninnere, d.h. den vorderen oder hinteren Augenabschnitt. Die Bioverfügbarkeit, d.h. der effektiven Transport des Wirkstoffes durch die Hornhaut (Cornea) oder Bindehaut/ Sklera hindurch ins Kammerwasser, respektive in den Glaskörper, an den Wirkort, wird durch verschiedene Faktoren beeinflusst:
- die physikochemischen Eigenschaften des Wirkstoffes und
- die Permeabilität der anatomischen Barriere
- präcorneale Faktoren wie die Tränensekretion oder der nasolakrimale Abfluss.

Das lipophile Epithel der Cornea bildet dabei die Hauptbarriere für hydrophile Wirkstoffe, weil die Zonulae occludentes mit den Tight junctions die Oberflächenzellen des Epithels derart umgeben, dass der parazelluläre Raum quasi versiegelt wird. Damit limitieren die Tight junctions die Permeation von Molekülen zwischen den Zellen.

Eine entsprechende Lipophilie der Arzneistoffe ist Voraussetzung für die transzelluläre Permeation im cornealen Epithel, wobei auch dabei sowohl die lipophile Zellmembran als auch das hydrophile Plasma passiert werden müssen.

Zur Verbesserung der Wirkstoffpenetration werden bei Augentropfen häufig quartäre Ammoniumverbindungen, wie Benzalkoniumchlorid, eingesetzt, die gleichzeitig auch als Konservierungsmittel wirken. Es ist belegt, dass Benzalkoniumchlorid die Hornhaut des Auges bis in die tieferen Zellschichten hinein angreift und schädigt. Auch andere Penetrationsenhancer wirken schädigend wie DMSO, Natriumglycocholat, Natriumfusidat, etc.

Somit war es Aufgabe der vorliegenden Erfindung, eine Zusammensetzung anzugeben, die einen effektiven Wirkstofftransport am Auge unter Vermeidung der o.g. Problematiken ermöglicht.

Diese Aufgabe wird bezüglich eines ophthalmologischen Vehikelsystems mit den Merkmalen des Patentanspruchs 1, bezüglich eines ophthalmologisches Kits mit den Merkmalen des Patentanspruchs 16 sowie bezüglich Verwendungsmöglichkeiten einer ophthalmologischen Zusammensetzung mit den Merkmalen des Patentanspruchs 17 gelöst. Mit Patentanspruch 18 wird ein Fluidspender, beinhaltend ein opthalmologisches Vehikelsystem gemäß der Erfindung angegeben. Die abhängigen Patentansprüche stellen dabei vorteilhafte Weiterbildungen dar.

Erfindungsgemäß wird somit ein ophthalmologisches Vehikelsystem zur Permeation und/oder zum Wirkstofftransport von ophthalmologischen Wirkstoffen durch die Hornhaut (Cornea) und/oder die Bindehaut (Sklera) des Auges eines Säugetieres bei der Prophylaxe und/oder Behandlung von Erkrankungen des vorderen und/oder hinteren Augenabschnittes bereitgestellt, enthaltend:
a) ≥ 30 bis 99,95 Gew.-%, bezogen auf die gesamte Zusammensetzung mindestens einen Fettsäureester,
b) 0,01 Gew.-% bis ≤ 50 Gew.-%, bezogen auf die gesamte Zusammensetzung einen oder mindestens einen Emulgator.

Das Auge kann in zwei Abschnitte untergliedert werden: den hinteren und den vorderen Augenabschnitt. Beide Abschnitte sind durch Linse und Regenbogenhaut räumlich voneinander getrennt. Die Begriffe vorderer und hinterer Augenabschnitt sind erfindungsgemäß wie folgt definiert:
vorderer Augenabschnitt:
   umfasst Bindehaut, Hornhaut, vordere Augenkammer, Iris und Linse.
hinterer Augenabschnitt:
   umfasst Ziliarkörper, Glaskörper, Netzhaut, Sehnervenkopf, Aderhaut und Lederhaut.

Die vorliegende Erfindung beschreibt eine Formulierung, die die Barrierefunktion der Hornhaut reversibel herabsetzt und so die effektive Permeation und Penetration eines Wirkstoffes durch die Cornea erhöht bzw. beschleunigt. Von großer Bedeutung ist, dass sich die Barrierefunktion der Cornea rasch wieder regeneriert und nicht irreversibel zerstört wird.

Die lipophile Basis der Formulierung bietet gegenüber wässrigen Formulierungen den Vorteil dass sie eine wesentlich längere Verweildauer im präcornealen Bereich aufweist und damit über längere Zeit Wirkstoff in das Auge abgibt.

Ein weiterer Vorteil ist die viskoelastische Eigenschaft der Formulierung, d.h. zwischen den Lidschlägen ist sie zähflüssiger (höher viskos), während des Lidschlags dünnflüssiger (niedriger viskos). Somit bildet sie einen gelartigen Schutzfilm auf der Augenoberfläche aus, der beim Lidschlag dünner wird, sich angenehm anfühlt und die Sicht nicht behindert.

Im Gegensatz zu den meisten lipophilen Formulierungen, wie Salben und Öle, die durch ihren hohen Brechungsindex ein verschwommenes Sehen verursachen, liegt der Brechungsindex der vorliegenden Formulierung mit circa 1,43 (je nach genauer Zusammensetzung) im Bereich der für Augenpräparate empfohlen ist (Siebenbrodt and Keipert, 1991).

Die Formulierung ist weiterhin nicht-irritierend und zeichnet sich durch eine hervorragende Verträglichkeit aus, was für Ophthalmika von besonderer Wichtigkeit ist.

Eine bevorzugte Ausführungsform sieht vor, dass der Fettsäureester ausgewählt ist aus der Gruppe bestehend aus Isopropylmyristat und Isopropylpalmitat.

Vorteilhaft ist ferner, wenn der Emulgator ein Lecithin oder eine Lecithinhaltige Zusammensetzung darstellt, bevorzugt ausgewählt ist aus Phosphatidylcholin-haltigen Zusammensetzungen mit einem Phosphatidylcholin-Gehalt von mindestens 90 Gew.-%, weiter bevorzugt mindestens 95 Gew.-%, insbesondere Epikuron 200 (Phosphatidylcholin mit ≥ 98 Gew.-%iger Reinheit) ist.

Eine besonders bevorzugte Ausführungsform der vorliegenden Erfindung sieht vor, dass lediglich ein einzelner Emulgator in der ophthalmologischen Zusammensetzung enthalten ist, insbesondere Phosphatidylcholin (Epikuron 200).

Eine weiter bevorzugte Ausführungsform sieht vor, dass der Gesamtgehalt des mindestens einen Fettsäureesters, bezogen auf die gesamte Zusammensetzung, von 50 bis 99,9 Gew.-%, bevorzugt 70 bis 99,9 Gew.-% beträgt.

Ebenso ist bevorzugt, wenn der Gesamtgehalt des einen oder mindestens einen Emulgators, bezogen auf die gesamte Zusammensetzung von 0,1 bis 15 Gew.-%, bevorzugt von 5 bis 12 Gew.-%, besonders bevorzugt von 7 bis 10 Gew.-% beträgt.

Zusätzlich kann das ophthalmologische Vehikelsystem gemäß der vorliegenden Erfindung mindestens eine ω-3-Fettsäure und/oder ein w-3-Fettsäure-derivat ausgewählt aus der Gruppe bestehend aus Estern, Mono-, Di- oder Triglyceriden, Lipiden, Oxygenierungsprodukten hiervon, wie z.B. Alkohole, Aldehyde, Ketone, Epoxide etc., Carboxylatsalzen, Amiden, sonstigen pharmakologisch akzeptablen Carbonsäurederivaten und Mischungen hiervon beinhalten. Beispiele für solche Omega-3 Fettsäure-Derivate sind die Resolvine (z.B. der Serie E und D, wie z.B. Resolvin E1 (RvE1) und Resolvin E2 (RvE2), sowie deren Isomere 18S-RvE1 und 18S-RvE2), Protectine (z.B. der Serie E und D, wie z.B.Protectin D1 und D2), Neuroprostane wie z.B. A4-NP, Isoprostane etc. bzw. deren synthetische Analoga.

Die mindestens eine w-3-Fettsäure kann dabei als freie Säure enthalten sein, aber auch in derivatisierter, d.h. abgewandelter Form vorliegen. Als w-3-Fettsäurederivate im Sinne der vorliegenden Erfindung sind sämtliche Verbindungen zu verstehen, die sich von einer w-3-Fettsäure ableiten oder eine w-3-Fettsäure als strukturelles Element beinhalten. Darunter zählen somit bspw. Salze der w-3-Fettsäuren, Stoffe, die eine kovalent gebundene w-3-Fettsäure aufweisen oder Stoffgemische, die eine w-3-Fettsäure als integralen Bestandteil beinhalten.

ω-3-Fettsäuren sind mehrfach ungesättigte Fettsäuren und gehören zu den essentiellen Fettsäuren, die in der Regel mit der Nahrung aufgenommen und im Körper in Zellmembranen eingebaut werden.

Die Folgeprodukte dieser Fettsäuren sind Gewebshormone und gelten als körpereigene, regulatorisch wirksame Stoffe. Sie beeinflussen zahlreiche Stoffwechselvorgänge und -funktionen.

In Abhängigkeit spezieller Stimuli, z.B. durch neutrale Reize oder sonstige Mediatoren, z.B. Histamine, werden w-3- und w-6-Fettsäuren aus den Membranlipiden freigesetzt und für die Biosynthese von diesen Gewebshormonen, den Eicosanoiden, zur Verfügung gestellt.

Diese wirken bereits in sehr geringen Konzentrationen (zwischen 10⁻⁸ und 10⁻¹⁰ mol pro Liter) als Mediatoren unmittelbar am Ort ihres Entstehens. Die Effekte werden entweder auf parakrinem Wege auf benachbarte Zellen ausgeübt oder auf autokrinem Wege auf die produzierende Zelle selbst. Die Reichweite dieser Mediatoren wird durch ihre sehr kurze Lebensdauer von Sekunden bis wenigen Minuten begrenzt. So wird auch bei topischer Applikation oder Inhalation ein günstiger Einfluss auf die Zusammensetzung genommen und eine lokal begrenzte Wirkung erreicht, was vorteilhaft für die therapeutische Anwendung ist. Daneben nimmt die Fettsäurezusammensetzung auch Einfluss auf die Permeabilität und Fluidität der Membranen.

Hierbei ist es bevorzugt, wenn die mindestens eine w-3-Fettsäure ausgewählt ist aus der Gruppe bestehend aus α-Linolensäure, Stearidonsäure, Eicosatetraensäure, Eicosapentaensäure (EPA), Docosapentaensäure (DPA), Docosahexaensäure, Hexadecatrienoensäure, Eicosatrienoensäure, Heneicosapentaenoensäure, Tetracosapentaenoensäure, Tetracosahexaenoensäure, hiervon abgeleitete Oxygenierungsprodukte, wie z.B. Alkohole, Aldehyde, Ketone, Epoxide etc. sowie Mischungen oder Kombinationen hieraus.

Ebenso ist es möglich, dass die mindestens eine w-3-Fettsäure in Form eines Esters eines organischen Alkohols, bevorzugt eines linearen oder verzweigten aliphatischen einwertigen Alkohols mit 1 bis 18 Kohlenstoffatomen, besonders bevorzugt als Methyl-, Ethyl-, n-Propyl-, i-Propyl-, n-Butyl-, i-Butyl-, t-Butylester enthalten ist.

Alternativ hierzu ist es ebenso denkbar, dass die mindestens eine w-3-Fettsäure in Form eines pflanzlichen oder tierischen Öls enthalten ist, das neben der mindestens einen w-3-Fettsäure noch mindestens eine w-6-Fettsäure enthält, wobei das molare Verhältnis der ω-3-Fettsäure zur ω-6-Fettsäure von 100:1 bis 1:100, bevorzugt 20:1 bis 1:10, weiter bevorzugt 15:1 bis 1:1, besonders bevorzugt von 8:1 bis 2:1 beträgt, enthalten ist.

Weiter ist es vorteilhaft, wenn die mindestens eine ω-3-Fettsäure in Form eines Öls, ausgewählt aus der Gruppe bestehend aus Algenöl, Fischöl, Perillaöl, Shiöl, Leinöl, Leindotteröl, Sacha Inchi Öl, Rapsöl, Olivenöl, Nachtkerzenöl, Sojaöl, Hanföl, Walnussöl, Erdnussöl, Sesamöl, Maiskeimöl, Flachssamenöl und/oder Mischungen hieraus vorliegt.

Ebenso sind auch Kombinationsmöglichkeiten aus Estern und Ölen der mindestens einen ω-3-Fettsäure denkbar.

Für den Fall, dass ω-3-Fettsäuren im ophthalmologischen Vehikelsystem enthalten sind, ist es von Vorteil, wenn der Gehalt der mindestens einen w-3-Fettsäure und/oder des Derivats hiervon, bezogen auf die gesamte Zusammensetzung, zwischen 0,01 und 49,9 Gew.-%, bevorzugt zwischen 0,05 und 30 Gew.-%, besonders bevorzugt zwischen 0,05 und 10 Gew.-% beträgt.

Weitere Vorteile ergeben sich, wenn die Zusammensetzung frei ist von Verbindungen, ausgewählt aus der Gruppe bestehend aus quartären Ammoniumverbindungen, insbesondere Benzalkoniumchlorid; DMSO, Natriumglycocholat und/oder Natriumfusidat. Vorteilhaft ist ebenfalls, wenn die Zusammensetzung frei von folgenden üblichen Salbengrundlagen zu halten: Paraffinen, Wollwachsen, Vaseline etc. ist.

Zudem ist es möglich, dass das ophtalmologische Vehikelsystem gemäß der vorliegenden Erfindung mindestens einen ophthalmologischen Wirkstoff in einer pharmazeutisch wirksamen Konzentration, insbesondere mindestens einen Wirkstoff ausgewählt aus der Gruppe bestehend aus Antibiotika, Corticoide, Lokalanästhetika, abschwellenden Medikamenten, nichtsteroidalen Antiphlogistika, Virustatica, Antiseptika, Kortison, antiallergischen Wirkstoffen, Prostaglandin-Analoga, Wirkstoffen aus der Wirkstoffklasse der Antihistaminika und/oder der Corticosteroide, antiallergischen Wirkstoffe, Pantothensäurederivaten, nichtsteroidalen Entzündungshemmer, Vaskokonstriktoren und/oder Anti-Glaucom-Wirkstoffen, Antiangiogenetische Wirkstoffe, (z.B. Aptamere (kurze einzelsträngige DNA- oder RNA-Oligonukleotide), Antikörper, o.ä.); Wirkstoffe zur Behandlung der Maculardegeneration enthält.

Denkbar ist, dass lediglich ein einzelner Wirkstoff enthalten ist, möglich sind jedoch ebenso Kombinationspräparate.

Der mindestens eine ophthalmologische Wirkstoff ist in diesem Falle bevorzugt zu 0,01 bis 40 Gew.-%, weiter bevorzugt zu .0,05 bis 20 Gew.-%, insbesondere von 0,1 bis 10 Gew.-%, bezogen auf das gesamte Vehikelsystem, enthalten. Die Art, die Anzahl sowie der genaue Gehalt des mindestens einen Wirkstoffs kann dabei jeweils spezifisch auf das jeweilige Anwendungsgebiet des Vehikelsystems abgestimmt werden.

Für einzelne ausgewählte Wirkstoffe gelten dabei die folgenden bevorzugten Konzentrationsbereiche:
Für Antibiotika, Virostatica, Corticoide, Kortison:
   Mindestens ein ophthalmischer Wirkstoff bevorzugt zu 0,01-10 Gew-%, weiter bevorzugt zu 0,05 - 5 Gew.-%, insbesondere von 0,1 - 3 Gew.-% bezogen auf das gesamte Vehikelsystem.
Antiallergika, Nichtsteroidale Entzündungshemmer:
   Mindestens ein ophthalmischer Wirkstoff bevorzugt zu 0,01 - 5 Gew-%, weiter bevorzugt zu 0,05 - 3 Gew.-%, insbesondere von 0,05 - 2 Gew.-% bezogen auf das gesamte Vehikelsystem.

Die Wirkstoffe können dabei ausgewählt sein aus natürlichen, synthetischen oder biotechnologisch hergestellten Wirkstoffen. Spezielle Beispiele hierfür werden nachfolgend angegeben:
Antibiotika:
   - Polypeptid-Antibiotika: Bacitracin, Polymyxin B, Gramicidin,
   - Aminoglykoside: Neomycin, Framycetin, Gentami-cin, Tobramycin,
   - Sulfonamide: Sulfacetamid,
   - Chinolone: Ciprofloxacin, Ofloxacin, Lomefloxacin, Moxifloxacin,
   - andere Antibiotika: Chloramphenicol Fusidinsäure
Alternativ alleine oder in Kombination mit okulären Glucocorticoiden
   - abschwellende Medikamente, wie Naphazolin, Phenylephrin, Tetryzolin, Tramazolin Xylometazolin;
   - nichtsteroidale Antiphlogistika, wie Diclofenac, Indometacin;
   - Virustatica, wie Aciclovir;
   - Antiseptika, wie Kortison, wie Hydrocortison, Rimexolon;
   - antiallergische Wirkstoffe aus der Antihistaminika, Corticosteroide, synthetische Mastzelldegranulationshemmer und Leukotrien-Rezeptor-Antagonisten;
   - Prostaglantin-Analoga, Antibiotika;
   - mindestens ein Wirkstoff aus der Wirkstoffklasse der Antihistaminika und/oder mindestens ein Wirkstoff aus der Wirkstoffklasse der Corticosteroide;
   - die Gruppe der Antihistaminika Ketotifen, Thonzylamin, Mepyramin, Thenalidin, Tripelenamin, Chlorpyramin, Promethazin, Tolpropamin, Dimetinden, Clemastin, Bamipin, Isothipendyl, Diphen-hydramin, Diphenhydraminmethylbromid, Chlorphe-noxamin, Pheniramin, Diphenylpyralin, Dioxopro¬methazin, Dimenhydrinat, Thiethylperazin und Meclozin, Azelastin, Levocabastin, Astemizol, Mebhydrolin, Terfenadin, Mequitazin, Cetirizin, Emedastin, Mizolastin, Olopatadin, Epinastin und Antazolin;
   - die Gruppe der Corticosteroide Triamcinolon, Dexamethason, Hydrocortison, Hydrocortison-acetat, Hydrocortisonbutyrat, Hydrocortisonbuteprat, Prednisolon, Betamethason, Methyl-prednisolon, Clobetason, Flumetason, Fluocortin, Fluperolon, Fluorometholon, Flupredniden, Desonid, Triamcinolon, Alclometason, Dexamethason, Clocortolon, Betamethason, Fluclorolon, Desoxi-metason, Fluocinolonacetonid, Fluocortolon, Diflucortolon, Fludroxycortid, Fluocinonid, Budesonid, Diflorason, Amcinonid, Halometason, Mometason, Methylprednisolonaceponat, Beclo-metason, Hydrocortisonaceponat, Fluticason, Prednicarbat, Prednison, Prednisolon, Diflu-prednat, Ulobetasol, Clobetasol, Halcinonid, Medryson, Desonid, Formocortal, Rimexolon, Mazipredon, Flunisolid und Tixocortol;
   - mindestens ein antiallergischer Wirkstoff aus der Gruppe Cromoglicinsäure, Spagluminsäure, Lodoxamid, Nedocromil, Montelukast und Zafirlukast;
   - Pantothensäurederivate Dexpanthenol, DL-Panthenol, Salze der Pantothensäure (z.B. Na-Pantothenat, Ca-Pantothenat), Ester der Pantothensäure (z.B. Ethyl-, Methylester), Panthenol-Ether (z.B. Ethyl-oder Methylether), Panthenol-Thioether sowie Panthenyltriacetat, besonders bevorzugt Dexpanthenol (= D-(+)-Pantothenyl¬alkohol);
   - alternativ nicht-steroidale Entzündungshemmer ("NSAIDs"), wie z.B. Aminoarylcarbonsäure-Derivate (z.B. Enfenaminsäure, Etofenamat, Flufenaminsäure, Isonixin, Meclofenaminsäure, Mefen¬aminsäure, Nifluminsäure, Talniflumat, Tero¬fenamat, Tolfenaminsäure), Arylacetalsäure-Derivate (z.B. Aceclofenac, Acemetacin, Alclofenac, Amfenac, Amtolmetinguacil, Bromfenac, Bufexamac, Cinmetacin, Clopirac, Diclofenacnatrium, Etodolac, Felbinac, Fenclozinsäure, Fentiazac, Glucametacin, Ibufenac, Indomethacin, Isofezolac, Isoxepac, lonazolac, Metiazinsäure, Mofezolac, Oxametacin, Pirazolac, Proglumetacin, Sulindac, Tiaramide, Tolmetin, Tropesin, Zomepirac), Aryl-Buttersäure-Derivate (z.B. Bumadizon, Butibufen, Fenbufen, Xenbucin), Arylcarbonsäure (z.B. Clidanac, Ketorolac, Tinoridin), Arylpropionsäure-Derivate (z.B. Alminoprofen, Benoxaprofen, Bermoprofen, Bucloxsäure, Carprofen, Fenoprofen, Flunoxaprofen, Flurbiprofen, Ibuprofen, Ibuproxam, Indoprofen, Ketoprofen, Loxoprofen, Naproxen, Oxaprozin, Piketoprolen, Pirofen, Pranoprofen, Protizinsäure, Suprofen, Tiaprofensäure, Ximoprofen, Zaltoprofen), Pyrazole (z.B. Difenamizol, Epirizol), Pyrazolone (z.B. Apazone, Benzpiperylone, Feprazone, Mofebutazone, Morazone, Oxyphenbutazone, Phenylbutazone, Pipebuzone, Propyphenazone, Ramifenazone, Suxibuzone, Thiazolinobutazone), Salicylsäure-Derivate (z.B. Acetaminosalol, Aspirin, Benorylate, Bromosaligenin, Calciumacetylsalicylate, Diflunisal, Etersalate, Fendosal, Gentisinsäure, Glycolsali-cylate, Imidazolsalicylate, Lysinacetylsalicylate, Mesalamine, Morpholinsalicylate, 1-Naphthylsalicylate, Olsalazine, Parsalmide, Phenyl-acetylsalicylate, Phenylsalicylate, Salacetamide, Salicylamide o Essigsäure, Salicylsulfonsäure, Salsalate, Sulfasalazine), Thiazincarboxamide (z.B. Ampiroxicam, Droxicam, Isoxicam, Lornoxicam, Piroxicam, Tenoxicam), ε-Acetamidcapronsäure, S (5'-adenosyl)-L-methionine, 3-amino-4-hydroxy-Buttersäure, Amixetrine, Bendazac, Benzydamine, α-Bisabolol, Bucolome, Difenpiramide, Ditazol, Emorfazon, Fepradinol, Guaiazulene, Nabumetone, Nimesulide, Oxaceprol, Paranyline, Perisoxal, Proquazone, Superoxid-Dismutase, Tenidap, Zileulon, deren physiologisch akzeptablen Salze sowie Kombinationen und Mischungen hiervon.

Andere nicht-steroidale Entzündungshemmer ("NSAIDs"), die zusätzlich in der erfindungsmäßigen Zusammensetzung enthalten sein können, umfassen Cyclooxygenase-Inhibitoren, z.B. selektive Inhibitoren der Cyclooxy-genase vom Typ II, wie z.B. Celecoxib und Etodolac, PAF (platelet activating factor)-Antagonisten, wie etwa Apafant, Bepafant, Minopafant, Nupafant, und Modipafant; PDE (Phosphodiesterase)-IV-Inhibitoren, wie etwa Ariflo, Torbafylline, Rolipram, Filaminast Piclamilast, Cipamfylline und Roflumilast; Inhibitoren der Cytokine-Bildung, wie etwa Inhibitoren des NF-κB-Transkriptionsfaktors; oder andere bekannte anti-entzündliche Agenzien.

Die erfindungsgemäße pharmazeutische Zu-sammensetzung kann aus der Gruppe der Vasokonstriktoren z.B. Oxy-metazolin, Xylometazolin, Tretryzolin, Naphazolin, Tramazolin und/oder deren Derivate als Wirkstoffkomponente enthalten.

Die erfindungsmäßige Zusammensetzung kann weiterhin Wirkstoffe mit antiangeogentischer Wirkung enthalten, z.B. VEGF-Inhibitoren z.B VEGF-Aptamere oder Antikörper, der als Arzneistoff zur Behandlung der altersabhängigen Makula-Degeneration (AMD) z.B. Macugen, Lucentis, Avastin u.a.

Zusätzlich zu den Fettsäuren können gegebenenfalls auch noch Anti-Glaucom-Wirkstoffe, wie
- Beta-Blocker: Timolol, Levobunolol,
- Cholinergika: Carbachol, Pilocarpin,
- Alpha-2-Adrenorezeptor-Agonist: Clonidin, Brimonidin, Carboanhydrasehemmer: Brinzolamid, Dor-zolamid und Acetazolamid,
- Prostaglandine: Latanoprost, Travoprost, Bimatoprost, Tafluprost, zugesetzt werden, um noch stärker auf die Wirkung Einfluss zu nehmen.

Die Konzentration der alternativ zugesetzten Agenzien, die in der vorliegenden Erfindung enthalten sind, kann je nach Agenz und Typ der Erkrankung variieren. Die Konzentration soll ausreichen, um z.B. eine Entzündung im behandelten Gewebe zu behandeln oder dieser vorzubeugen. Typischerweise liegen die Konzentrationen dabei im Bereich von 0,0001 bis etwa 5 % wt/wt (oder alternativ bei 0,01 bis etwa 2 % wt/wt, oder von etwa 0,05 % bis 1 % wt/wt, oder von etwa 0,01 % bis etwa 0,5 % wt/wt).

Ebenso können Antikörper, Aptamere, si RNA oder andere "small molecules" zur Vorbeugung oder Behandlung von Erkrankungen des Auges enthalten sind.

Ebenso kann können zusätzlich Modulatoren, z.B. Inhibitoren, Antagonisten, insbesondere Modulatoren des NF-κB-Transkriptionsfaktors enthalten sein.

Immunologische Prozesse, Prozesse in Entzündungsverläufen und Wundheilungsprozesse bilden ein eng verwobenes Zusammenspiel bei Reizungen, Entzündungen und Heilungsvorgängen und sind untrennbar miteinander verknüpft. Modulatoren sind Stoffe, die in dieses komplexe Zusammenspiel regulierend/modulierend einwirken und so die optimale Funktion des Immunsystems unterstützen und/oder einen positiven Effekt auf die Prophylaxe oder Behandlung von Reizungen, Entzündungen und/oder die Wundheilung ausüben.

Folgende Modulatoren physiologischer Prozesse, wie Reizungen, Entzündungsverläufe und/oder Wundheilung, sind aus dem Stand der Technik bekannt und vom oben verwendeten Begriff Modulator umfasst:
- Coenzym Q10 (Q10, Ubichinon-10). Q10 ist essentiell auf mitochondrialer Ebene zur optimalen Funktion des Immunsystems (Folkers K, Wolaniuk A; Research on coenzyme Q10 in clinical medicine and in immunomodulation. Drugs Exp Clin Res. 1985;11(8):539-45). Q10 wirkt in entzündlichen Prozessen auf der Ebene der Genexpression. Es übt u.a. anti-inflammatorische Effekt über Einfluss auf NFkappaB1-abhängige Gen-Expression aus (Schmelzer C, Lindner I, Rimbach G, Niklowitz P, Menke T, Döring F., Functions of coenzyme Q10 in inflammation and gene expression, Biofactors 2008;32(1-4):179-83). Q10 kann in einer Konzen-tration von 1-100 μm, bevorzugt in einer Konzentration von 2-10 μM, verwendet werden;
- Taurin. Taurin kommt in Immunzellen vor und moduliert bestimmte Immunzellfunktionen, so z.B. Regulation von inflammatorischen Aspekten der Immunantwort. Es wirkt auch als Schutz in seiner Funktion als Antioxidants (Fazzino F, Obregön F, Lima L. Taurine and proliferation of lymphocytes in physically restrained rats. J Biomed Sci. 2010 Aug 24;17 Suppl 1:S24) und als Osmoregulator (Shioda R., Reinach P.S., Hisatsune T., Miyamoto Y. Osmosensitive taurine transporter expression and activity in human corneal epithelial cell. IOVS, Sept. 2002, Vol. 43, No. 9). Taurin kann in einer Konzentration von 0,1-50 mM, bevorzugt 0,1-5 mM, verwendet werden;
- Carboxymethylcellulose (CMC). CMC bindet an humane Epithelzellen und ist ein Modulator der kornealen epithelialen Wundheilung (Invest Ophthalmol Vis Sci. 2007 Apr;48(4):1559-67). CMC-Bindung an die Matrix stimuliert die Anheftung, Migration und Reepithelialisierung kornealer Wunden in HCEC;
- Resolvin (insbesondere die E- und D-Serie). Resolvin E1 (RvE1) induziert eine Zunahme der Zell-Migration und damit eine beschleunigte epitheliale Wundheilung (Zhang et al, IOVS, Vol. 51, No. 11, November 2010); und
- Protectine. Protectine sind, wie Resolvine, Derivate der Eicosapentaensäure und Docosahexaensäure üben anti-inflammatorische Effekte aus (Curr Opin Clin Nutr Metab Care. 2011 Mar;14(2):132-7.Docosahexaenoic acid, protectins and dry eye. Cortina MS, Bazan HE)

Überraschenderweise wurde gefunden, dass mit dem erfindungsgemäßen Vehikelsystem bei Prophylaxe und/oder Behandlung von Erkrankungen des vorderen und/oder hinteren Augenabschnittes zusätzlich die Entstehung von Gewebereizungen und -schädigungen von Allergien oder Entzündungen effizient vorgebeugt und vermieden werden kann. Ferner kann bei bereits aufgetretenen Schädigungen in das Entzündungsgeschehen eingegriffen werden, wobei sich eine Verbesserung der Heilung ggf. vorhandener Verletzungen oder Wunden des Augenepithels erzielen lässt.

Wenn Gewebereizungen oder -schädigungen erfolgen, sei es durch Umweltnoxen, mechanische Reize, wie Reibung, Druck, durch Bakterien, Trauma, Chemikalien, Hitze und/oder überschießende Immunreaktionen, wie Allergien, etc., kommt es zunächst zu Aktivitätsänderungen in bestimmten zellulären Signalwegen, die wiederum zu spezifischen Änderungen des Genexpressionsmusters führen.

Es werden in den betroffenen Geweben diverse Entzündungsmediatoren freigesetzt, die Entzündungsprozesse einleiten und aufrechterhalten. Die Gesamtheit dieser komplexen Gewebeveränderungen wird als Entzündung bezeichnet. Reguliert ablaufende Entzündungen spielen bei den Prozessen der Wundheilung eine wichtige Rolle.

Das bevorzugte, modulatorhaltige Vehikelsystem greift zur Vermeidung, Modulation oder Hemmung auf verschiedenen Ebenen in diese komplexen Vorgänge ein:
Durch das erfindungsgemäße Vehikelsystem in Kombination mit einem Modulator, z.B. einem Inhibitor, Antagonist, etc., des NF-κB-Transkriptionsfaktors einsetzt, wird die Entstehung entzündungshemmender Mediatoren begünstigt, um prophylaktisch oder therapeutisch Entzündungen vorzubeugen, so dass eine weitere Verbesserung der Abheilung bestehender Verletzungen des Auges begünstigt wird, da Entzündungen, die die Abheilung negativ beeinflussen, unterdrückt werden können. Ebenso wurde jedoch auch die Abheilung bei bereits bestehenden Entzündungen beobachtet.

Einer der wichtigsten intrazellulären Regulatoren von Entzündungsreaktionen ist der Transkriptionsfaktor NF-κB, der durch verschiedene Formen von Zellstress aktiviert wird, etwa chemisch-physikalische Noxen, bakterielle und virale Antigene, Zytokine, etc., und die Genexpression in betroffenen Zellen schnell und umfassend ändern kann. Unter den Genen, die entsprechend hoch reguliert werden, befinden sich insbesondere Zytokine, wie IL-1, TNFalpha, Enzyme, wie COX-2, iNOS, Zelladhäsionsmoleküle, etc., die für eine Ausbreitung der Entzündungsreaktion auf andere Zellen und deren Verstärkung, oft im Sinne einer positiven Rückkopplung, sorgen.

Die Modulation der Aktivierung von NF-κB stellt eine weitere Möglichkeit dar, mit der das erfinderische Vehikelsystem in das Entzündungsgeschehen eingreifen kann.

Modulatoren des NF-kappa B Transkriptionsfaktors können z.B. direkt auf NF-kappa B wirken, oder indirekt über die Signalkaskade auf NF-kappa B. Antioxidantien z.B. können Komponenten des NF-kappa B Signaltransduktionsweges inhibieren, einschließlich des TNF Rezeptors und des Proteasoms.

Durch die Modulation von NF-kappa B kann sich eine Modulation (insbesondere Suppression) des Tumor-Nekrose-Faktors alpha (TNF-α) ergeben. TNF-α ist ein Signalstoff des Immunsystemes und spielt bei Erkrankungen wie z.B. beim Sjögren-Syndrom, der Keratoconjunctivitis sicca, Erkrankungen der Meimom-Drüse eine große Rolle.

Während des Entzündungsprozesses werden große Mengen an reaktiven Sauerstoff- und Stickstoffspezies gebildet, die u.a. auch regulatorisch in den Entzündungsprozess eingreifen. So wird etwa das Superoxid-Radikal in Immunzellen, wie Monozyten, Makrophagen und polymorphkernigen Leukozyten, durch membranständige NADPH-Oxidasen gebildet und in das extra-zelluläre Milieu abgegeben. In durch entzündliche Stimuli aktivierten Zellen steigt die normalerweise nur geringe Superoxid-Bildung um das Zehnfache an ("oxidative burst"). Die Bildung dieser Spezies ist nicht nur für die Abtötung der Bakterien verantwortlich, sondern dient auch der Rekrutierung von Leukozyten zum Entzündungsherd und hat somit eine Funktion zur Entzündungsverstärkung. Weiterhin greifen reaktive Sauerstoffspezies auf der Ebene der Transkription in die Bildung von Enzymen, z.B. NOS-II, ein. Sie aktivieren auch Transkriptionsfaktoren, wie z.B. die NF-κB-B-Familie und Proteinkinasen, während sie Protein-Tyrosin-Phosphatasen inaktivieren.

Auch die reaktive Stickstoffverbindung Stickstoffmonoxid wird enzymatisch und streng kontrolliert in einer Reihe von Geweben gebildet. Ausgangssubstanz ist die Aminosäure L-Arginin, aus der das freie Radikal durch das Enzym NO-Synthestase (NOS) gebildet wird. In Immunzellen, speziell in Makrophagen und Granulozyten, kann nach Stimulation die induzierbare NOS (iNOS) exprimiert werden. Stimuli sind dabei vor allem Auslöser von Entzündungsreaktionen, wie Bakterien oder deren Bestandteile, inflammatorische Zytokine etc.

Das Quenchen und Abfangen solcher reaktiven Sauerstoff- und Stickstoffspezies kann die Reaktionskette unterbrechen und so regulatorisch auf Transkriptions- und Enzymaktivierungsebene wirken. Zusätzlich können Gewebeschäden, wie z.B. Lipidperoxidationen, durch reaktive Sauerstoffspezies vermieden werden.

Eine prophylaktische oder therapeutische Wirkung erfolgt somit auf verschiedenen Ebenen des Prozesses.

So werden durch die Ausbildung einer Art Schutzschicht z.B. mechanischen Reizungen oder der Kontakt mit Allergenen oder Bakterien vermieden. Das erfindungsgemäße Vehikelsystem enthält daher bevorzugt entsprechend z.B. lipophile und Gel-bildende Komponenten, Konsitenzgeber, Viskositätserhöher, die einen befeuchtenden, pflegenden Film auf Epithelien, wie etwa der Cornea, ausbilden können. Beispiele für Gelbildner in Oleogelen sind u.a. die Ethylene/Propylene/Styrene Copolymer Butylene/Ethylene/Styrene Copolymer, hochdisperses SiO₂, Al-stearat, Zn-stearat, Agar Agar (und) Alginsäure.

Beispiele für Konistenzgeber sind u.a. Castoröl, Jojobaöl, Cetylpalmitat, Sheabutter/ Kakaobutter, Oleyloleat.

Weiterhin greifen Komponenten des beschriebenen modulatorhaltigen Vehikelsystems auf einer oder mehreren Ebenen, z.B. Gentranskription, qualitative und/oder quantitative Modulation der Freisetzung von Mediator-Molekülen, Modulation der Signaltransduktion, etc., in z.B. den Prozess der Entzündungsentstehung und/oder Verstärkung regulierend ein.

Durch die Auswahl und die Zusammensetzung der w-3-Fettsäuren im Vehikelsystem kann die Entstehung entzündungshemmender Mediatoren begünstigt werden, um prophylaktisch oder therapeutisch Störungen und Erkrankungen vorzubeugen. Beispiele für Erkrankungen sind Irritationen, Reizung und Schwellung der Schleimhäute, Augenbrennen, Wundheilung, Behandlung des Keratokonjunktivitis sicca, des Sjörgen-Syndroms.

In einem bevorzugten Vehlkelsystem ist der mindestens eine Modulator ein Inhibitor oder Antagonist des NF-κB-Transkriptionsfaktors und bevorzugt ausgewählt
- aus natürlichen Quellen, insbesondere aus der Gruppe bestehend aus Allicin, Curcumin, EGCD, Genistein, Melatonin, Quercetin, Resveratrol, Silymarin, Sulphoraphanen oder Mischungen hieraus, und/oder
- aus der Gruppe bestehend aus synthetischen Inhibitoren, insbesondere Pyrrolidin-dicarbamat, 2-Chlor-4-(trifluormethyl)pyrimidin-5-N-(3',5'-bis(trifluor-methyl)phenyl)-carboxamid und/oder Mischungen hieraus.

Weitere Beispiele für Modulatoren der NF-kB-Aktivierung (Antagonisten und/oder Inhibitoren) aus natürlichen Quellen sind: alpha-Liponsäure (Thioctsäure) und Dihydroliponsäure, 2-Amino-1-methyl-6-phenylimidazol (4,5 b]pyridine (PhIP), N-acetyldopamindimere (from P. cicadae), Allopurinol, Anetholdithiolthione, Apocynin, Aretemsia p7F (5,6,3',5'-tetramethoxy 7,4'-hydroxyflavone), Astaxanthin, Autumn olive extracts; olive leaf extracts, Avenanthramide (from oats), Bamboo culm extract, Benidipin, Bis-eugenol, Bruguiera gymnorrhiza compounds, butyliertes Hydroxyanisol (BHA), Cepharanthin, Caffeic Acid Phenethyl Ester (3,4-dihydroxycinnamic acid, CAPE), Carnosol, Carotinoide (z.B. beta-Carotin), Carvedilol, Catechol Derivatives, Centaurea L (Asteraceae) extracts, Chalcone, Chlorogenic acid, 5-chloroacetyl-2-amino-1,3-selenazoles, Cholestin, Chroman-2-carboxylic acid N-substituted phenylamides, polyphenols for example from Cocoa or Crataegus pinnatifida, Coffee extract (3-methyl-1,2-cyclopenta-nedione), Curcumin (Diferulolylmethan); dimethoxycur-cumin; ER24 anlog, Dehydroepiandrosterone (DHEA) und DHEA-Sulfat (DHEAS), Dibenzylbutyrolactone lignans, Diethyldithiocarbamat (DDC), Diferoxamin, Dihydroisoeugenol; Isoeugenol; Epoxypseudoisoeugenol-2-methylbutyrat, Dihydrolipoic Acid, Dilazep + feno-fibric acid, Dimethyldithiocarbamates (DMDTC), Disulfiram, Edaravone, EPC-K1 (phosphodiester compound of vitamin E and vitamin C) Epigallocalechin-3-gallate (EGCG; green tea polyphenols), Ergothioneine, Ethylene Glycol Tetraacetic Acid (EGTA), Eupatilin, Fisetin, Flavonoids (Crataegus; Boerhaavia diffusa root; xanthohumol; Eupatorium arnottianum; genistein; kaempferol; quercetin, daidzein; flavone; isorhamnetin; naringenin; pelargonidin; finestin; Sophora flavescens; Seabuckthorn fruit berry), Sesquiterpenlactone wie z.B. Helenalin z.B aus Arnika-Extrakten, Folic acid, Gamma-glutamylcysteine synthetase (gamma-GCS), Ganoderma lucidum polysaccharides, Garcinol (from extract of Garcinia indica fruit rind), Ginkgo biloba extract, Glutathione, Guaiacol (2-methoxyphenol), Hematein, Hinokitiol, Hydroquinone, 23-hydroxyursolic acid, IRFI 042 (Vitamin E-like compound), Iron tetrakis, Isoflavone, Isosteviol, Isovitexin, Isoliqui¬ritigenin, Kallistatin, Kangen-karyu extract, L-cysteine, Lacidipine, Lazaroids, Ligonberries, Lupeot, Lutein, Magnolol, Maltol, Melatonin, Extract ofthe stem bark of Mangifera indica L., 21(alpha, beta)-methylmelianodiol, 21(alpha,beta)-methylmeli-anodiol, Mulberry anthocyanins, N-acetyl-L-cysteine (NAC), Nacyselyn (NAL), Nordihydroguaiaritic acid (NDGA), Ochnaflavone, Onion extract (2,3-dihydro-3,5-dihydroxy-6-methyl-4H-pyranone), Orthophenanthroline, N-(3-oxo-dodecanoyl)homoserine lactone, N-(3-oxo-dodecanoyl)homoserine lactone, Paricalcitol, Parthe¬nolid, ein Sesquiterpenlacton, Terpene und ein Sesquiterpenlacton, Parthenolid, Phenolic antioxidants (Hydroquinone and tert-butyl hydroquinone), alkenylphenols from Piper obliquum, alpha-phenyl-n-tert-butyl-njtrone (PBN), Phenylarsine oxide (PAO, tyrosine phosphatase inhibitor), Phyllanthus urinaria, Phytosteryl ferulates (rice bran), Piper longum Linn. Extract, Pitavastatin Prodelphinidin B2 3,3'di-O-gallate, Pterostilbene, Pyrrolinedithiocar-bamate (PDTC), Quercetin, Ref-1 (redox factor 1), Ref-1 (redox factor 1), Rotenone, Roxithromycin, Rutin, S-allyl-cysteine (SAC, garlic compound), Salo-gaviolide (Centaurea ainetensis), Sauchinone, Silybin, Spironolactone, Taxifolin, Tempol, Tepoxaline (5-(4-chlorophenyl)-N-hydroxy(4-methoxyphenyl)-N-methyl-1H-pyrazole-3-propanamide), Thymoquinone, Tocotrienol (palm oil), Vanillin (2-hydroxy-3-methoxybenzaldehyde), Vitamin B6, a-torphryl succinate, a-torphryl succinate, 2-torphryl acetate, PMC (2,2,5,7,8-pentamethyl-6-hydroxychromane), Yakuchinone A and B, Zerumbon aus Zingiberarten (Ingwer).

Als weitere Beispiele für synthetische Quellen sind folgende zu nennen: Kortisone und Glukokortikoide, sowie deren Ester, z.B. 16α,17-[(R)-Cyclohexyl-methy¬lendioxy]-11β,21-dihydroxypregna-1,4-dien-3,20-dion-21-isobutyrat), Salicylanilid-Inhibitoren, 3,4-dihydro-1,1-dimethyl-2H-1, 2-benzoselenazine; Declopramide und Dexlipotam, N-(-Acetylphenyl)-2-hydroxy-5-iodphenyl-carboxamid; N-(-2,4-Difluoro¬phenyl)-2-hydroxy-5-nitrophenylcarboxamid; N-(2,4-Difluorphenyl)-2 hydroxy-5-iodphenylcarboxamid, oder ein pharmazeutisch annehmbares Salz, Hydrat oder Solvat davon.

Es können auch zusätzlich Modulatoren der COX-2 enthalten sein, wie z.B. Basilikum, Berberine, Curcumin, EGCG, Ingwer, Hopfen (Humulus lupulus), Fischöl, Oregano, Quercetin, Resveratrol, Rosemarie.

Besonders bevorzugte Wirkstoffe, die im erfindungsgemäßen ophthalmologischen Vehikelsystem eingesetzt werden sind entzündungshemmende Stoffe, wie Acetylsalicalsäure und Derivate, z.B als L-Lysin, antiseptische Stoffe, wie Biprocathol, Antibiotika wie Ampicillin, Sulfacetamid, Doxycyclin, Gentamycin oder Ciprofloxacin, Antiallergika wie Cromoglicinsäure, Antihistaminika wie Levocabastin, Azelastin und/oder Dexpanthenol.

Das erfindungsgemäße ophthalmologische Vehikelsystem ist bevorzugt konservierungsmittelfrei. Für den Fall, dass Konservierungsmittel enthalten sind, ist es besonders bevorzugt und ausreichend, dass lediglich Silberionen Vehikelsystem vorliegen. Die Silberionen können dabei in einem bevorzugten Konzentrationsbereich von 1 ppb bis 2 ppm, weiter bevorzugt von 10 ppb bis 1 ppm enthalten sein. Die Anwesenheit der Silberionen kann durch den externen Zusatz von Silbersalzen, wie z.B. Silbernitrat zum Vehlkelsystem resultieren, aber auch dadurch resultieren, dass das Vehikelsystem mit einem Gegenstand aus einer Silberlegierung oder massivem Silber kontaktiert wird, wodurch sich ein geringer Anteil des Silbers auflöst und in das Vehikelsystem übergeht. Etwaige Silber enthaltenden Gegenstände können z.B. Silberspiralen, wie sie in Dosiervorrichtungen für Flüssigkeiten oder Pasten vorkommen, sein. Entsprechende Dosiervorrichtungen sind z.B. aus den Patentanmeldungen EP 1466 668 A1 bekannt. Die o.g. geringen Silberkonzentrationen sind ausreichend, um einen konservierenden Effekt, z.B. einen bakteriziden Effekt auf das Vehikelsystem auszuüben.

Insbesondere für den Fall, dass,das Vehikelsystem Silberionen enthält, oder angedacht ist, das Vehikelsystem mit einem Silber haltigen Gegenstand in Kontakt zu bringen, ist es vorteilhaft, dem Vehikelsystem mindestens einen Komplexbildner, ausgewählt aus der Gruppe der schwefelhaltigen organischen Verbindungen, insbesondere Natriumthiosalicylsäure, Thiosorbit, Cystein, N-Acetyl-L-cystein, Cyteinhydrochlorid, Cysteamin, Cystin, Methionin, Glutathion, S-Acetylglutathion, Thioglycerol, Thioharnstoff, Thiolactat; und/oder EDTA, EGTA sowie Kombinationen hieraus, bevorzugt in einer Menge von 0,0001 - 5 Gew.-%, vorzugsweise 0,0001-1 Gew.-%, bevorzugt 0,001-0,5 Gew.-%, bezogen auf das gesamte Vehikelsystem zuzusetzen.

Alternativ hierzu kann das Vehikelsystem jedoch ebenso Konservierungsstoffe enthalten, z.B. mindestens ein in der Ophthalmologie gebräuchliches Konservierungsmittel in einer Menge von 0,001- 1 Gew.-%, vorzugsweise 0,01-0,5 Gew.-%, bevorzugt 0,01-0,04 Gew.-%, bezogen auf die gesamte Zusammensetzung, vorzugsweise ein Konservierungsmittel ausgewählt aus der Gruppe bestehend aus Polyquad, Natriumperborat, Purite; Alkohole, wie Chlorobutanol, Benzylalkohol, Phenoxyethanol; Carboxylsäuren wie Sorbinsäure; Phenole, wie Methly-/ Ethylparaben; Amidine wie Chorhexidindigluconat; quaternäre Ammoniumverbindungen wie Benzalkoniumchlorid, Benzethoniumchlorid und Cetylpyridiniumchlorid, Benzylbromid und/oder Kombinationen hieraus.

Das Vehikelsystem gemäß der vorliegenden Erfindung kann zudem einen oder mehrere der folgenden Bestandteile beinhalten:
a) mindestens mindestens einen anti-inflammatorisch und/oder antioxidativ und/oder antiallergisch wirkenden Stoff" ausgewählt aus der Gruppe bestehend aus Flavonoiden (z.B. Rutin, Quercetin, Curcurmin), Isoflavonoiden (z.B. Silymarin), Polyphenole (z.B. Resveratol), Anthocyanen, Triterpenen, Monoterpenalkoholen, Phenolcarbonsäuren, Carotinoiden (z.B. β-Carotin, α-Carotin, Lycopin, β-Cryptoxanthin, Lutein, Zeaxanthin), Retinoide (z.B. Tretinoin), Tocopherolen (Vitamin E) und Biotin, Vitamine A, C, D, K, Coenzym Q (=Q10) Carnitin, N-Acetyl-Carnitin, Glutathion, Carnesol, Ubichinon und/oder Taurin und/oder pflanzliche Einzelstoffe, Stoffgemische, ein flüssiger oder fester Extrakt, ein Destillat oder ein Öl oder ätherisches Öl, bevorzugt aus Pflanzen der Gattungen oder Arten Rosmarin, Sanddorn, Myrrhe, Eupharis (Augentrost), Kamille, Arnika, Ringelblume, Thymian, Echina, Calendula, Teebaum, Teestrauch, Apfelbeere (Aronia), Ginkgo, Ginseng, Heidelbeere, Holunder, Lavendel, Anis, bevorzugt in einer Menge von 0,01 und 5 Gew.-%, bezogen auf die gesamte Zusammensetzung,
b) mindestens einen Gelbildner, ausgewählt aus der Gruppe bestehend aus natürlichen oder synthetischen Polymeren, bevorzugt in einer Menge zwischen 0,01 und 5 Gew.-%, bezogen auf die gesamte Zusammensetzung,
c) mindestens ein Verdickungsmittel, bevorzugt in einer Menge zwischen 0,5 und 5 Gew.-%, bezogen auf die gesamte Zusammensetzung.
d) mindestens ein Feuchthaltemittel,
e) mindestens eine Hilfsstoff, ausgewählt aus der Gruppe bestehend aus anorganischen Puffersubstanzen, organischen Puffersubstanzen, anorganischen Salzen, organischen Salzen, Viskositätsreglern, Lösungsmitteln, Lösungsvermittlern(z.B. Lecithin, Macrogolglycerolmonosterat, Macrogolglycerolricenoleat, Polyethylenemonosterate (z.B. Myrj 49), Polysorbate, Glycerylmonooleate, Glycerylmonosterate, Glyceryllaurat, Methylcellulose, Polychol, Sorbitanmonolaurat, Sorbitanoleat, Sorbitanpalmitat, Sorbitantrioleat,u.a
f) Lösungsbeschleunigern, Salzbildnern, Viskositäts- und Konsistenzbeeinflussern, Solubilisatoren, Benetzern, Spreizmitteln, Füll- und Trägerstoffen, Osmolaritätsreglern sowie Mischungen davon, sowie
g) Kombinationen aus den zuvor genannten Bestandteilen.

Der mindestens eine anti-inflammatorisch und/oder antioxidativ und/oder antiallergisch wirkenden Stoff wird in erster Linie wegen seiner physiologischen Wirkung im Auge eingesetzt. Diese Stoffe unterbinden z.B. oxidative Schäden im Gewebe z.B. hervorgerufen durch reaktive Sauerstoffspezies bei überschießenden Immunreaktionen; sie wirken z.T. entzündungshemmend, etc. Zudem schützen sie auch Omega-3 FS vor Oxidation in der Formulierung vor oxidativem Abbau.

Hierbei umfasst Vitamin A das Vitamin A1 (Retinol), Vitamin A2 (3-Dehydroretinol), Vitamin-A-Säure, Vitamin-A-Abkömmlinge (Retinylpalmitat, Retinylacetat etc.), all-trans-Retinolsäure (ATRA, aRA, Tretinoin), 13-cis-Retinolsäure bzw. -Retinsäure (Isotretinoin), Vitamin A-Analoge wie z.B. die all-trans Retinsäure.

Vitamin C umfasst Ascorbinsäure, Ascorbylpalmitat und Ascorbylacetat und Vitamin E umfasst Gamma-Tocotrienol und 6-hydroxy-2,5,7,8-tetramethyl-chroman-2-carboxylsäure (Trolox).

Beispiele für Antioxidantien sind Terpenoide (Monoterpenoid, Sesquiterpenoid, Diterpenoid, Triterpenoid), Carotinoide (α- und β-Carotin), Hydroxy-tyrosol, Zeathin, Lutein, Lycopene, Anthocyanine, Cryptoxan-thine, Xanthophylle, Epicatechin, Quercetin, Punicalagine und Ellagsäure; Chlorogensäure, Gallensäure, Ferulasäure, Kaffeesäure, a-Tocopherol, a-Tocopherolester, Ascorbinsäure, Ascorbinsäureester ( myristat, -palmitat und -stearat), ß-Carotin, Cystein, Acetylcystein (N-Acetyl-L-cystein stellt auch gleichzeitig ein mucolytisches Mittel dar), Coenzym Q, Idebenone (synthetisches Quinone ähnlich Q10), Folsäure (Vitamin-B2-Gruppe), Phytinsäure, cis-und/oder trans-Uro¬cansäure, Karnosin (N-ß-Alanin-L-Histidin), Histidin, Flavone, Flavonoide, Lycopin, Taurin, Tyrosin, Gluthation, Gluthationester, α-Liponsäure, Ubichinon, Niacin, Nordihydroguaiaretsäure, Gallussäureester (Ethyl-, Propyl-, Octyl-, Dodecylgallat), Phosphorsäurederivate (Monophosphate, Polyphosphate), Butylhydroxytoluol, Butylhydroxyanisol, Tetraoxydimethylbiphenyl, Tocotrienole (Teil der Vitamin-E-Stoffgruppe), Polyalkohole, Polyphenole, Zitronensäure, Weinsäure, Edetinsäure (EDTA als DiNa- oder DiNaCa-Salz), Coniferylbenzoat und/oder deren Derivate als Antioxidationsmittel.

Die Antioxidantien können direkt oder auch in Form von Ölen oder ätherischen Ölen zugesetzt werden.

Weizenkeimöl enthält z.B. Tocopherole, Karotinoide, Ergocalciferol, Folsäure (Vitamin B9), Panthoten-säure, Phytosterine und Phenole, wie Dihydroquercetin, etc.

Für das erfindungsgemäße Vehikelsystem geeignete Gelbildner umfassen vorzugsweise natürliche oder synthetische Polymere. Natürliche Polymere sind vorzugsweise ausgewählt aus der Gruppe bestehend aus Agar-Agar, Alginsäure, Alginat, amidiertes Pektin, Propylenglycolalginat, Carbomer, Carrageenan, Casein, Cellulose-Derivate (Methyl-, Hydroxyethyl, Carboxymethylcellulose-Natrium), Dammar-Gummi, Dextrine, Furcellaran, Gelatine, Guargummi, Guarkernmehl, Gellan, Ghatti-Gummi, Gummi arabicum, Gummi aus Fichtensaft, Johannisbrotkernmehl, Karaya-Gummi, Keratin, Konjakmehl, L-HPC, Locust Bean Gum, Mastix, Pektin, Schellack, (ggf. modifizierte) Stärke, Tarakernmehl, Traganth, Xanthangummi und deren Derivate Verdickungsmittel die vorzugsweise im erfindungsgemäßen Vehikelsystem enthalten sein können, umfassen beispielsweise Candelilla und Carnaubawachs sowie mikrokristalline Wachse, Carbomer, Polyethylenoxid-Verdicker, Polaxamere, Hydroxyethylcellulose, Hydroxypropylcellulose, Hypromellose, Povidon, Hyaluronsäure, Polymilchsäure und Derivate davon. Das Verdickungsmittel wird vorzugsweise in einer Menge von 0,5 bis 5 Gew.-%, bezogen auf das Gesamtgewicht der erfindungsgemäßen, pharmazeutischen Zusammensetzung, eingesetzt.

Bevorzugte Formulierungen, in denen das ophtalmologische Vehikelsystem der vorliegenden Erfindung vorliegen kann, sind dabei in flüssiger, viskoser oder halbfester Form, insbesondere in Form eines Gels, eines thixotropen Gels, eines Lipogels, eines Oleogels, eines Organogels, eines Mikroemulsionsgels, eines Sprühgels, einer Wasser-in-Öl-Emulsion, oder eines in situ Gels.

Mikroemulsionsgele sind dabei Emulsionsgele, bei denen der mittlere Teilchendurchmesser d₅₀ kleiner als 0,1 μm ist.

Das ophthalmologische Vehikelsystem der vorliegenden Erfindung eignet sich insbesondere zur Prophylaxe und/oder Behandlung von Entzündung (z.B. Uveitis (Uveitis anterior, Uveitis intermedia, Uveitis posterior, Panuveitis), Iritis, Chorioiditis, Azoor (Acute zonal occult outer retinopathy/Akute zonale okkulte äußere Retinopathie), Neuritis nervi optici), Katarakt (Grauer Star), Glaucom, Retinopathie, Makuladegenerationen (AMD), Netzhautablösung, Retinoblastom und/oder Aderhautmelanom und/oder zur Vor- und/oder Nachbehandlung chirurgischer Eingriffe am Auge, insbesondere chirurgischer Eingriffe ausgewählt aus der Gruppe bestehend aus chirurgischen Eingriffen am vorderen Augenabschnitt, Kataraktextraktion mit Linsenimplantation, refraktiv-chirurgischen Eingriffen, Eingriffen an der Hornhaut und Hornhauttransplantationen und/oder Eingriffen an der Bindehaut. Applikationsmöglichkeiten des ophthalmologischen Vehikelsystems sind dabei insbesondere die topische Applikation am Auge, z.B. durch Einträufein oder Eintropfen ins Auge bzw. auf die Augenoberfläche, Einsprühen oder Aufsprühen ins Auge bzw. auf die Augenoberfläche, oder durch Einträufeln oder eintropfen, als Geldepot in den/ im Bindehautsack oder als Insert.

Bevorzugt kann das Vehikelsystem der vorliegenden Erfindung 1 mal täglich bis 1mal stündlich, bevorzugt 1 bis 4 mal täglich appliziert werden.

Zudem betrifft die vorliegende Erfindung ein ophthalmologisches Kit, umfassend
a) ein zuvor beschriebenes ophthalmologisches Vehikelsystem sowie
b) eine ophthalmologische Wirkstoffformulierung
   als separate Formulierungen.

Das ophthalmologische Kit gemäß der vorliegenden Erfindung eignet sich dabei für die gleichen Indikationen wie das zuvor beschriebene ophthalmologische Vehikelsystem. Im Unterschied zum ophthalmologischen Vehikelsystem besteht das Kit aus mindestens zwei separaten Bestandteilen, d.h. Formulierungen, die beispielsweise gleichzeitig miteinander, aber auch nacheinander prinzipiell in beliebiger Reihenfolge, bevorzugt aber das Vehikelsystem zuerst, am Auge appliziert werden können. Dabei sind die Applikationsmöglichkeiten ebenso identisch mit dem mit denen für das zuvor beschriebene ophthalmologische Vehikelsystem.

In einer bevorzugten Ausführungsform ist das ophthalmologische Vehikelsystem, als Bestandteil des ophthalmologischen Kits, frei von Wirkstoffen, d.h. der Wirkstoff wird im Kit separat und zusätzlich zum ophthalmologischen Vehikelsystem bereitgestellt. Diese ermöglicht eine große Applikationsbandbreite, sodass eine besonders zielgerichtete Applikation der einzelnen Wirkstoffe am Auge zusammen mit dem ophthalmologischen Vehikelsystem zur Verbesserung der Permeation des Wirkstoffs erzielt werden. Daneben können optimale Lagerbedingungen für die einzelnen Formulierungen separat gewählt werden. So kann das ophthalmologische Vehikelsystem bei bevorzugten Lagerbedingungen, daneben ebenso die ophthalmologische Wirkstoffformulierungen bei optimalen Lagerbedingungen hierfür aufbewahrt werden, sodass insgesamt eine lange Haltbarkeit des gesamten ophthalmologischen Kits gewährleistet werden kann.

Ebenso ist denkbar, dass das ophthalmologische Vehikelsystem als Bestandteil des Kits bereits einen oder mehrere ophthalmologische Wirkstoffe enthält, wobei mit der zusätzlichen ophthalmologischen Wirkstoffformulierung noch mindestens ein weiterer Wirkstoff hinzu kombiniert werden kann.

Die Bestandteile des Kits können z.B. unmittelbar vor Applikation am Auge miteinander vermischt und das Gemisch auf die weiter oben beschriebenen bevorzugten Applikationsarten am oder ins Auge appliziert werden; ebenso ist jedoch auch die einzelne Aufgabe der Komponenten des Kits am oder ins Auge möglich.

Gegenstand der vorliegenden Erfindung ist ebenso die Verwendung einer Zusammensetzung, enthaltend
a) ≥ 50 bis 99,9 Gew.-%, bezogen auf die gesamte Zusammensetzung mindestens einen Fettsäureester,
b) 0,1 Gew.-% bis ≤ 50 Gew.-%, bezogen auf die gesamte Zusammensetzung einen oder mindestens einen Emulgator
als Vehikelystem, Penetrationsbeschleuniger, Penetrationsenhancer, Absorptionsenhancer/-verbesserer/-beschleuniger zur Permeation und/oder zum Wirkstofftransport von ophthalmologischen Wirkstoffen durch die Hornhaut (Cornea) und/oder die Bindehaut (Sklera) des Auges eines Säugetieres.

Dabei wird die zuvor beschriebene Zusammensetzung auf die gleiche Art und Weise und/oder für die gleichen Anwendungsmöglichkeiten, wie zuvor für das ophthalmologische Vehikelsytem bzw. das ophthalmologische Kit beschrieben, angewandt. Die bevorzugten stofflichen Zusammensetzungen der oben beschriebenen verwendeten Zusammensetzung entsprechen dabei ebenso denjenigen des ophthalmologischen Vehikelsystems, wie oben beschrieben.

Die Erfindung betrifft zudem einen Fluidspender für ein keimfreies Fluid, mit
a) einem Durchgang, der eine Einlassöffnung für ein in einem Vorratsbehälter aus einem flexiblen Material enthaltenes Fluid und eine Ausströmöffnung zum Ausgeben des Fluids verbindet und darin wenigstens eine oligodynamisch aktive Substanz besitzt, die mit dem Fluid in Kontakt ist;
b) einer Dosierpumpe, die ohne Luftdruckkompensation arbeitet, umfassend ein Einlassventil zum Schließen der Einlassöffnung, wobei das Einlassventil ein Material aufweist, das mit dem Fluid über eine oligodynamisch aktive Substanz wechselwirken kann; und
c) eine Federeinrichtung, die in Kontakt mit dem Fluid steht,
   wobei das Einlassventil und die Federeinrichtung ein rostfreies Stahlmaterial als eine oligodynamisch aktive Substanz aufweisen und eine Dekontaminierungseinrichtung im oberen Teil des Auslasskanals vorgesehen ist, wobei die Dekontaminierungseinrichtung ein Material aufweist, das mit dem Fluid über eine oligodynamische Substanz wechselwirken kann, die ausgewählt ist aus der Gruppe bestehend aus Silber, Silbersalzen, anderen Silberverbindungen, Legierungen und Nanomeren davon in entweder metallischer oder Salzform oder als eine chemische Verbindung daraus, wobei das im Vorratsbehälter enthaltene Fluid ein erfindungsgemäßes ophthalmologisches Vehikelsystem ist.

Bezüglich des mi dem Fluidspenders, in dem gemäß der Erfindung das ophthalmologische Vehikelsystem bevorratet werden kann wird auf die Patentanmeldung EP 1466 668 A1 verwiesen. Sämtliche Ausführungsformen bezüglich des Fluidspenders dieser Patentanmeldung werden durch Bezugnahme auch zum Gegenstand dieser Patentanmeldung gemacht.

Die vorliegende Erfindung wird anhand der nachfolgenden beispielhaften Formulierungen näher erläutert, ohne die Erfindung auf die dort dargestellten speziellen Parameter zu beschränken.

Die folgenden beispielhaften erfindungsgemäßen Rezepturen eignen sich als ophthalmologisches Vehikelsystem zur Permeation und/oder zum Wirkstofftransport von ophthalmologischen Wirkstoffen durch die Hornhaut (Cornea) und/oder die Bindehaut (Sklera) des Auges eines Säugetieres zur Prophylaxe und/oder Behandlung von Erkrankungen des vorderen, und/oder hinteren

Augenabschnittes.

### Beispiel 1- Transparente Mikroemulsionsgel-Grundlage zur Formulierung verschiedener Wirkstoffe

| **Bestandteile** | **Menge (Gew.-%)** |
|---|---|
| Epikuron 200 | 7,5 % |
| Isopropylmyrisat | 92 % |
| DHA/ EPA | 0,1 % |
| *alternativ auch Q10* | *0,05* % |
| Wasser | 0,4 % (bis zur Gelbildung |

### Beispiel 2 - Transparente Mikroemulsionsgel-Grundlage zur Formulierung verschiedener Wirkstoffe

| **Bestandteile** | **Menge (Gew.-%)** |
|---|---|
| Epikuron 200 | 7,5 % |
| Isopropyl*palmitat* | 92 % |
| DHA/ EPA | 0,1 % |
| *alternativ auch Q10* | *0,05 %* |
| Wasser | 0,4 % (bis zur Gelbildung |

### Beispiel 3 - Transparente Mikroemulsionsgel-Grundlage zur Formulierung verschiedener Wirkstoffe

| **Bestandteile** | **Menge (Gew.-%)** |
|---|---|
| Epikuron 200 | 7,5 % |
| Isopropylmyristat | 87,05 % |
| Algenöl | 5 % |
| *alternativ auch Q10* | *0,05 %* |
| Wasser | 0,4 % (bis zur Gelbildung |

### Beispiel 4 - Ölige Grundlage zur Formulierung verschiedener Wirkstoffe

| **Bestandteile** | **Menge (Gew.-%)** |
|---|---|
| Epikuron 200 | 3 % |
| Isopropylmyristat | 96,45 % |
| Rapsöl | 0,5 % |
| *alternativ auch Tocopherol* | *0,05 %* |

### Beispiel 5 - Ölige Grundlage zur Formulierung verschiedener Wirkstoffe

| **Bestandteile** | **Menge (Gew.-%)** |
|---|---|
| Epikuron 200 | 0,5 % |
| Isopropylmyristat | 99,175% |
| DHA/ EPA | 0,2 % |
| Ascorbinsäurepalmitat | 0,05 % |
| Tocopherol | 0,075 % |

### Beispiel 6 - Ölige Grundlage zur Formulierung verschiedener Wirkstoffe

| **Bestandteile** | **Menge (Gew.-%)** |
|---|---|
| Lecithin | 5 % |
| Isopropylpalmitat | 74,5 % |
| Castoröl | 15% |
| Carnesol | 0,05 % |
| Triglyceroldiisosterat | 1% |

### Beispiel 7 - Ölige Grundlage zur Formulierung verschiedener Wirkstoffe

| **Bestandteile** | **Menge (Gew.-%)** |
|---|---|
| Lecithin | 1 % |
| Isopropylpalmitat | 74,5 % |
| Castoröl | 50 % |
| Carnesol | 0,05 % |
| Triglyceroldiisosterat | 1% |

Die Wirksamkeit des erfindungsgemäßen ophthalmologischen Vehikelsystems wird durch die nachfolgenden Untersuchungen belegt.

### I. EVEIT Test

Die nachfolgenden Experimente belegen das Permeationsvermögen der erfindungsgemäßen ophthalmologischen Vehikelsystems. Hierbei wurde als beispielhafte, ins Auge einzubringende Substanz Fluoreszein untersucht. Die eingesetzten Substanzen sind dabei:
Kulturmedium:
   Ringerlösung: Die Ringer-Lösung ist eine isotone Elektrolytlösung, die u.a. als Nährmedium für Frischgewebe verwendet wird. Die Standard-Ringerlösung enthält auf 1000 ml Aqua destillata 8,6 g Natriumchlorid, 0,3 g Kaliumchlorid, 0,33 g Calciumchlorid.
Hylo-COMOD^{®}:
   HYLO-COMOD^{®} ist eine sterile, konservierungsmittelfreie Lösung mit 1 mg/ml Natriumhyaluronat, einem Citratpuffer, Sorbitol und Wasser und wird von der URSAPHARM Arzneimitel GmbH vertrieben.
Ursapharm A:
   Mikroemulsionsgel auf Wasser-in-Öl-Basis, enthaltend 7,5 Gew.-% Lecithin (Epicuron 200), 0,4 Gew.-% Wasser, 0,1 Gew.-% eines Gemischs aus Eicosapentaensäureethylester / Docosapentaensäureethylester (Gewichtsverhältnis ca. 73 : 27) sowie ad 100 Gew.-% Isopropylmyristat
Ursapharm C:
   Mikroemulsionsgel auf Öl-in-Wasser-Basis, enthaltend 0,1 Gew.-% eines Gemischs aus Eicosapentaensäureethylester / Docosapentaensäureethylester (Gewichtsverhältnis ca. 73 : 27), 1 Gew.-% Magrogolglycerolricinoleat (z.B. Cremophor EL), 0,1 Gew.-% Hyaluronsäure, < 0,3 Gew.-% gemischte Tocopherole, 0,05 Gew.-% Q10, 3,2 Gew.-% Sorbitol, 0,005 Gew.-% Citronensäure, 0,85 Gew.-% Natriumcitrat sowie ad 100 Gew.-% Wasser.

### Experiment 1: Negativkontrolle - lediglich Simulation des Lidschlages durch Applikation von Kulturmedium

Präparation und Kultivierung einer Kaninchenhornhaut nach dem EVEIT System. Die Präparation erfolgte maximal 8h post mortem. Die Kultivierung erfolgte bei 32° C und einer Luftfeuchtigkeit von > 95 %. Eine Simulation des Lidschlages erfolgte durch Applikation von Kulturmedium (38μl) auf den Hornhautapex in einem Zeitintervall von 60 min. 24h nach Präparation wurde die Kultur einer Qualitätsuntersuchung unterzogen (makroskopisch inkl. Fluorescein-Färbung sowie durch optische Kohärenztomographie (OCT)), die eine erfolgreiche Inkulturnahme mit physiologischer Vitalität der Organkultur belegt. Im Anschluss wurde die Hornhaut über einen Beobachtungszeitraum von 3 Tagen unter Beibehaltung der Applikation von Kulturmedium alle 60 Minuten weiterkultiviert. Die Dokumentation des Versuchsverlaufes erfolgte täglich durch Makroskopie und OCT. Die Hornhautkulturen wurden täglich einmal für 60 Sekunden mit 100μl einer wässrigen Lösung von Natrium Fluorescein (5 mg/ml) inkubiert. Anschließend wurde das Fluorescein von der Hornhautoberfläche mit Ringerlösung gründlich abgespült. Nach dem Abspülen wurden die Hornhautkulturen für 60 Minuten im Brutschrank bei 32° C und 100 % Luftfeuchtigkeit stehen gelassen. In diesem Zeitfenster erfolgte keinerlei Austausch des Kulturmediums in der Vorderkammer. Das Vorderkammervolumen wurde nach den 60 Minuten Wartezeit vollständig ausgetauscht. Die zu diesem Zeitpunkt in der künstlichen Vorderkammer befindliche Stoffmenge Fluorescein wurde photometrisch bestimmt.

Ergebnis: In der OCT ist innerhalb der Kulturzeit keinerlei strukturelle Änderung von Epithel oder des Stroma erkennbar. Im Verlauf der Kultur ist 48 Stunden nach Erstapplikation eine Quellung der Hornhaut zu beobachten. Diese Zunahme der Schichtdicke im Bereich von 30 % ist eine typische Beobachtung innerhalb der Kultur die im physiologischen Bereich liegt und lediglich auf eine etwas erhöhte Wasseraufnahme des Stromas unter den Kulturbedingungen hindeutet. Makroskopisch liegt ebenfalls keinerlei Auffälligkeit vor. Die grüne Färbung der Makroskopien wird durch geringe Mengen an Fluorescein in der künstlichen Vorderkammer verursacht, welche aus den parallel durchgeführten Permeationsmessungen resultieren. Die Epithelien selbst sind Fluorescein-negativ.

### Experiment 2: Tropfapplikation alle 60 min, HYLO-COMOD^{®}

Präparation und Kultivierung einer Kaninchenhornhaut nach dem EVEIT System. Die Präparation erfolgte maximal 8h post mortem. Die Kultivierung erfolgte bei 32° C und einer Luftfeuchtigkeit von > 95 %. Eine Simulation des Lidschlages erfolgte durch Applikation von Kulturmedium (38μl) auf den Hornhautapex in einem Zeitintervall von 60 min. 24h nach Präparation wurde die Kultur einer Qualitätsuntersuchung unterzogen (makroskopisch inkl. Fluorescein-Färbung sowie durch optische Kohärenztomographie (OCT)), die eine erfolgreiche Inkulturnahme mit physiologischer Vitalität der Organkultur belegt. Im Anschluss daran wurde die Testsubstanz HYLO-COMOD^{®} in einem Zeitintervall von 60 min wiederholt über einen Zeitraum von 3 Tagen auf die Hornhaut aufgebracht (38μl). Das Aufbringen der Testsubstanz erfolgte dabei im 30-minütigen Wechsel mit der Applikation des Kulturmediums. Die Dokumentation des Versuchsverlaufes erfolgte täglich durch Makroskopie und OCT. Die Hornhautkulturen wurden täglich einmal für 60 Sekunden mit 100µl einer wässrigen Lösung von Natrium Fluorescein (5 mg/ml) inkubiert. Anschließend wurde das Fluorescein von der Hornhautoberfläche mit Ringerlösung gründlich abgespült. Nach dem Abspülen wurden die Hornhautkulturen für 60 Minuten im Brutschrank bei 32° C und 100 % Luftfeuchtigkeit stehen gelassen. In diesem Zeitfenster erfolgte keinerlei Austausch des Kulturmediums in der Vorderkammer. Das Vorderkammervolumen wurde nach den 60 Minuten Wartezeit vollständig ausgetauscht. Die zu diesem Zeitpunkt in der künstlichen Vorderkammer befindliche Stoffmenge Fluorescein wurde photometrisch bestimmt.

Ergebnis: In der OCT ist innerhalb der Kulturzeit keinerlei strukturelle Änderung des Epithels aufgrund der Applikation erkennbar. Im Verlauf der Kultur ist 48 Stunden nach Erstapplikation eine Quellung der Hornhaut zu beobachten. Diese Zunahme der Schichtdicke ist eine typische Beobachtung innerhalb der Kultur die im physiologischen Bereich liegt und lediglich auf eine etwas erhöhte Wasseraufnahme des Stromas unter den Kulturbedingungen hindeutet. Makroskopisch liegt ebenfalls keinerlei Auffälligkeit vor. Die grüne Färbung der Makroskopien wird durch geringe Mengen an Fluorescein in der künstlichen Vorderkammer verursacht, welche aus den parallel durchgeführten Permeationsmessungen resultieren. Alle Epithelien sind Fluorescein-negativ.

### Experiment 3: Positivkontrolle - Tropfapplikation alle 60 min, Benzalkoniumchlorid 0,001 % in Ringer-Lösung

Präparation und Kultivierung einer Kaninchenhornhaut nach dem EVEIT System. Die Präparation erfolgte maximal 8h post mortem. Die Kultivierung erfolgte bei 32° C und einer Luftfeuchtigkeit von > 95 %. Eine Simulation des Lidschlages erfolgte durch Applikation von Kulturmedium (38µl) auf den Hornhautapex in einem Zeitintervall von 60 min. 24h nach Präparation wurde die Kultur einer Qualitätsuntersuchung unterzogen (makroskopisch inkl. Fluorescein-Färbung sowie durch optische Kohärenztomographie (OCT)), die eine erfolgreiche Inkulturnahme mit physiologischer Vitalität der Organkultur belegt. Im Anschluss daran wurde die Testsubstanz BAC 0,001 % in einem Zeitintervall von 60 min wiederholt über einen Zeitraum von 3 Tagen auf die Hornhaut aufgebracht (38µl). Das Aufbringen der Testsubstanz erfolgte dabei im 30-minütigen Wechsel mit der Applikation des Kulturmediums. Die Dokumentation des Versuchsverlaufes erfolgte täglich durch Makroskopie und OCT. Die Hornhautkulturen wurden täglich einmal für 60 Sekunden mit 100µl einer wässrigen Lösung von Natrium Fluorescein (5 mg/ml) inkubiert. Anschließend wurde das Fluorescein von der Hornhautoberfläche mit Ringerlösung gründlich abgespült. Nach dem Abspülen wurden die Hornhautkulturen für 60 Minuten im Brutschrank bei 32° C und 100 % Luftfeuchtigkeit stehen gelassen. In diesem Zeitfenster erfolgte keinerlei Austausch des Kulturmediums in der Vorderkammer. Das Vorderkammervolumen wurde nach den 60 Minuten Wartezeit vollständig ausgetauscht. Die zu diesem Zeitpunkt in der künstlichen Vorderkammer befindliche Stoffmenge Fluorescein wurde photometrisch bestimmt.

Ergebnis: In der OCT ist 48 Stunden nach Erstapplikation von BAC 0,001 % eine deutliche Abnahme der Epithelschichtdicke erkennbar. 72h nach Erstapplikation hat sich das Epithel strukturell nahezu aufgelöst und bildet keine geschlossene Schicht mehr. Aufgrund dieses Epitheldefektes liegt die Quellung der Hornhaut zu diesem Zeitpunkt deutlich über dem sonst unter physiologischen Bedingungen beobachteten Maß. Die grüne Färbung der Makroskopien wird teilweise durch geringe Mengen an Fluorescein in der künstlichen Vorderkammer verursacht, welche aus den parallel durchgeführten Permeationsmessungen resultieren. 48 Stunden und 72 Stunden nach Erstapplikation ist das Epithel jedoch deutlich Fluorescein-positiv.

### Experiment 4: Tropfapplikation alle 60 min, Ursapharm A (Lecithin-Gel + IPM 0,4 %)

Präparation und Kultivierung einer Kaninchenhornhaut nach dem EVEIT System. Die Präparation erfolgte maximal 8h post mortem. Die Kultivierung erfolgte bei 32° C und einer Luftfeuchtigkeit von > 95 %. Eine Simulation des Lidschlages erfolgte durch Applikation von Kulturmedium (38µl) auf den Hornhautapex in einem Zeitintervall von 60 min. 24h nach Präparation wurde die Kultur einer Qualitätsuntersuchung unterzogen (makroskopisch inkl. Fluorescein-Färbung sowie durch optische Kohärenztomographie (OCT)), die eine erfolgreiche Inkulturnahme mit physiologischer Vitalität der Organkultur belegt. Im Anschluss daran wurde die Testsubstanz Ursapharm A in einem Zeitintervall von 60 min wiederholt über einen Zeitraum von 3 Tagen auf die Hornhaut aufgebracht (38µl). Das Aufbringen der Testsubstanz erfolgte dabei im 30-minütigen Wechsel mit der Applikation des Kulturmediums. Die Dokumentation des Versuchsverlaufes erfolgte täglich durch Makroskopie und OCT. Die Hornhautkulturen wurden täglich einmal für 60 Sekunden mit 100µl einer wässrigen Lösung von Natrium Fluorescein (5 mg/ml) inkubiert. Anschließend wurde das Fluorescein von der Hornhautoberfläche mit Ringerlösung gründlich abgespült. Nach dem Abspülen wurden die Hornhautkulturen für 60 Minuten im Brutschrank bei 32° C und 100 % Luftfeuchtigkeit stehen gelassen. In diesem Zeitfenster erfolgte keinerlei Austausch des Kulturmediums in der Vorderkammer. Das Vorderkammervolumen wurde nach den 60 Minuten Wartezeit vollständig ausgetauscht. Die zu diesem Zeitpunkt in der künstlichen Vorderkammer befindliche Stoffmenge Fluorescein wurde photometrisch bestimmt.

Ergebnis: Im Versuch ist die Hornhaut über den Versuchszeitraum deutlich anschwellend mit einer Anfangsdicke von 405 auf 649 µm. Der Epithelverband ist am Ende nicht mehr nachweisbar. Dementsprechend findet sich in der Fluoresceinfärbung eine vollständige zentrale Anfärbung der Hornhaut. Das Bild entspricht der Positivkontrolle.

### Experiment 5: Tropfapplikation alle 60 min, Ursapharm C

Präparation und Kultivierung einer Kaninchenhornhaut nach dem EVEIT System. Die Präparation erfolgte maximal 8h post mortem. Die Kultivierung erfolgte bei 32° C und einer Luftfeuchtigkeit von > 95 %. Eine Simulation des Lidschlages erfolgte durch Applikation von Kulturmedium (38µl) auf den Hornhautapex in einem Zeitintervall von 60 min. 24h nach Präparation wurde die Kultur einer Qualitätsuntersuchung unterzogen (makroskopisch inkl. Fluorescein-Färbung sowie durch optische Kohärenztomographie (OCT)), die eine erfolgreiche Inkulturnahme mit physiologischer Vitalität der Organkultur belegt. Im Anschluss daran wurde die Testsubstanz Ursapharm C in einem Zeitintervall von 60 min wiederholt über einen Zeitraum von 3 Tagen auf die Hornhaut aufgebracht (38µl). Das Aufbringen der Testsubstanz erfolgte dabei im 30-minütigen Wechsel mit der Applikation des Kulturmediums. Die Dokumentation des Versuchsverlaufes erfolgte täglich durch Makroskopie und OCT. Die Hornhautkulturen wurden täglich einmal für 60 Sekunden mit 100µl einer wässrigen Lösung von Natrium Fluorescein (5 mg/ml) inkubiert. Anschließend wurde das Fluorescein von der Hornhautoberfläche mit Ringerlösung gründlich abgespült. Nach dem Abspülen wurden die Hornhautkulturen für 60 Minuten im Brutschrank bei 32° C und 100 % Luftfeuchtigkeit stehen gelassen. In diesem Zeitfenster erfolgte keinerlei Austausch des Kulturmediums in der Vorderkammer. Das Vorderkammervolumen wurde nach den 60 Minuten Wartezeit vollständig ausgetauscht. Die zu diesem Zeitpunkt in der künstlichen Vorderkammer befindliche Stoffmenge Fluorescein wurde photometrisch bestimmt.

Ergebnis: In der OCT ist innerhalb der Kulturzeit keinerlei strukturelle Änderung des Epithels aufgrund der Applikation erkennbar. Im Verlauf der Kultur ist 48 Stunden nach Erstapplikation eine Quellung der Hornhaut zu beobachten. Diese Zunahme der Schichtdicke ist eine typische Beobachtung innerhalb der Kultur, die im physiologischen Bereich liegt und lediglich auf eine etwas erhöhte Wasseraufnahme des Stromas unter den Kulturbedingungen hindeutet. Makroskopisch liegt ebenfalls keinerlei Auffälligkeit vor. Die grüne Färbung der Makroskopien wird durch geringe Mengen an Fluorescein in der künstlichen Vorderkammer verursacht, welche aus den parallel durchgeführten Permeationsmessungen resultieren. Alle Epithelien sind Fluorescein-negativ.

### II. Quantitative Auswertung der Fluorescein-Permeation in die künstliche Vorderkammer unter Tropfapplikation

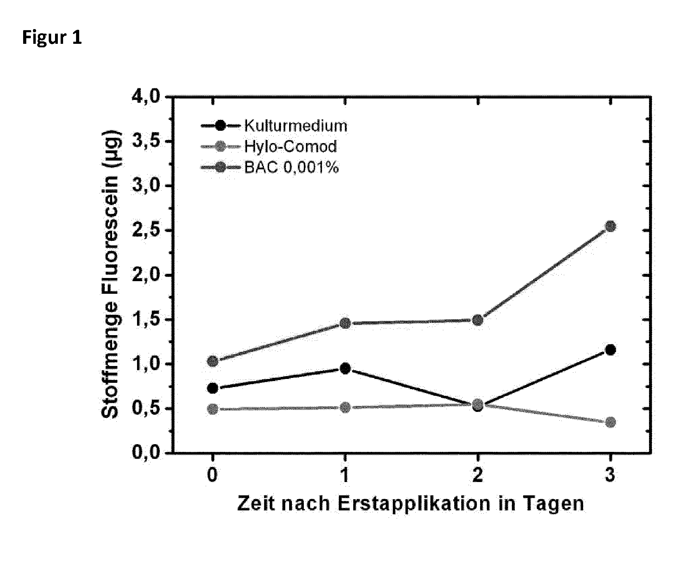

### Fluorescein-Permeation: Kontrollsubstanzen (Kulturmedium (Experiment 1), HYLO-COMOD^{®} (Experiment 2) und Benzalkoniumchlorid 0,001 % (Experiment 3); Figur 1)

In **Figur 1** ist die festgestellte Stoffmenge an Fluorescein innerhalb der simulierten Augenvorderkammer über die Zeit nach Erstapplikation der aufgeführten Kontrollsubstanzen aufgetragen. Der Ausgangswert am Zeitpunkt null wurde direkt vor Erstapplikation gemessen. Im Anschluss wurde alle 24 Stunden ein weiterer Wert ermittelt. Wird auf das Hornhautepithel lediglich Kulturmedium in einem Zeitintervall von 60 Minuten zur Simulation des Lidschlages aufgetropft, so steigt die Permeabilität der Hornhaut über die Kulturdauer von 3 Tagen um etwa 50 % an. Wird zwischen der Simulation des Lidschlages zusätzlich HYLO-COMOD^{®} auf die Hornhaut aufgetropft, so kann dieser Anstieg vollständig unterdrückt werden. Wird anstelle von HYLO-COMOD^{®} der Permeations-Enhancer Benzalkoniumchlorid (0,001 %) im Wechsel mit der Applikation mit Kulturmedium auf die Hornhaut aufgebracht, so steigt die gemessene Permeation von Fluorescein deutlich um mehr als 150 Prozent an. Die Untersuchung zeigt an, dass es eine geringe Permeation von Fluorescein durch das Epithel zu allen Zeitpunkten des EVEIT gibt, durch chemische Alteration mittels des Konservierungsmittels Benzalkoniumchlorid, welches als Detergenz wirkt und die epitheliale Integrität der zonulae occludentes verändert, lässt sich ein Permeations Anstieg, der kontinuierlich über die Versuchszeit zunimmt, nachweisen. Dies zeigt den kumulativen und weitgehenden Schaden an, welcher durch Benzalkoniumchlorid in der Langzeitapplikation bewirkt wird.

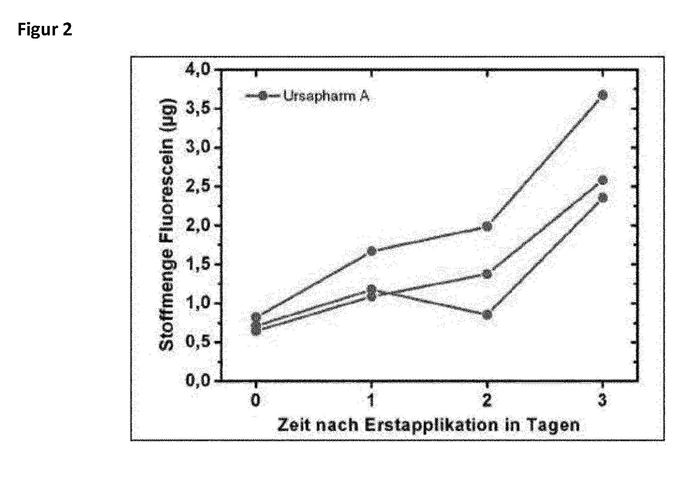

### Fluorescein-Permeation: Ursapharm A (Lecithin-Gel + IPM 0,4 %; 3 Datensätze (Experiment 4), Figur 2)

In **Figur 2** ist die festgestellte Stoffmenge an Fluorescein innerhalb der simulierten Augenvorderkammer über die Zeit nach Erstapplikation der Testsubstanz Ursapharm A (Lecithin-Gel + IPM 0,4 %) aufgetragen. Der Ausgangswert am Zeitpunkt null wurde direkt vor Erstapplikation gemessen. Im Anschluss wurde alle 24 Stunden ein weiterer Wert ermittelt. Wird zwischen der Simulation des Lidschlages (durch Auftropfen von Kulturmedium) zusätzlich die Testsubstanz Ursapharm A (Lecithin-Gel + IPM 0,4 %) auf die Hornhaut aufgebracht, so ist ein deutlicher Anstieg der Permeation von Fluorescein mit der Zeit zu beobachten. Dieser Anstieg ist nach 24 Stunden bereits signifikant. Im Applikationsintervall zwischen 48 und 72 Stunden nach Erstapplikation ist der Anstieg am steilsten. Dies entspricht dem beobachteten Verhalten des bekannten Permeations-Enhancers Benzalkoniumchlorid. Der relative Anstieg der Fluoresceinpermeation innerhalb der Beobachtungszeit von 3 Tagen liegt im Bereich von 280 bis 450 Prozent und ist damit höher als der beobachtete Wert für Benzalkoniumchlorid (0,001 %). Dieses Ergebnis zeigt eine massive Beschädigung der epithelialen Integrität, welche bereits im makroskopischen wie auch histologischen Bild imponiert. Der Verlust der Flügelzellen und die praktisch exponierte Basalzellschicht bieten keinen Schutz vor dem eindringenden Farbstoff. Damit ist das Epithel als geschädigt zu betrachten.

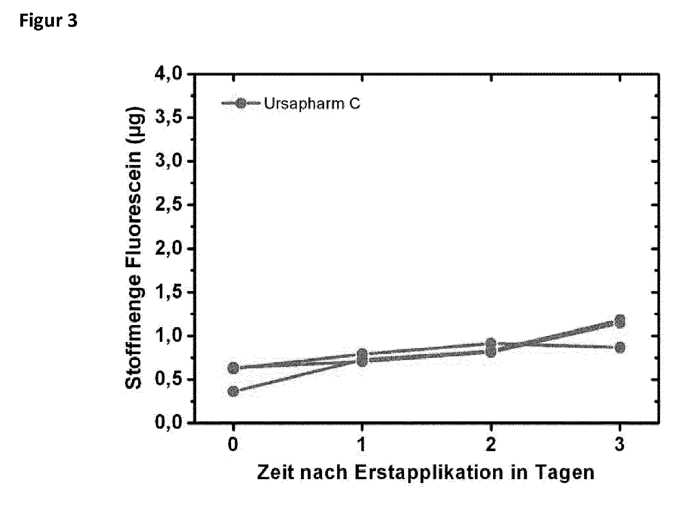

### Fluorescein-Permeation: Ursapharm C (3 Datensätze (Experiment 5), Figur 3)

Aufgetragen ist in **Figur 3** die festgestellte Stoffmenge an Fluorescein innerhalb der simulierten Augenvorderkammer über die Zeit nach Erstapplikation der Testsubstanz Ursapharm C. Der Ausgangswert am Zeitpunkt null wurde direkt vor Erstapplikation gemessen. Im Anschluss wurde alle 24 Stunden ein weiterer Wert ermittelt. Wird zwischen der Simulation des Lidschlages (durch Auftropfen von Kulturmedium) zusätzlich die Testsubstanz Ursapharm C auf die Hornhaut aufgebracht, so ist ein signifikanter aber geringer Anstieg der Permeation von Fluorescein mit der Zeit zu beobachten. Dieser Anstieg entspricht im Umfang dem Anstieg, der unter üblichen Kulturbedingungen ohne die Applikation einer Testsubstanz ebenfalls beobachtet wird. Die Epitheliale Integrität erscheint unverletzt. Die im histologischen Bild gesehenen Hyperplasien der anterioren Epithelverbände haben keinen negativen Einfluss auf die Integrität der zonulae occludentes, welche die Permeation des Farbstoffes sicher verhindern.

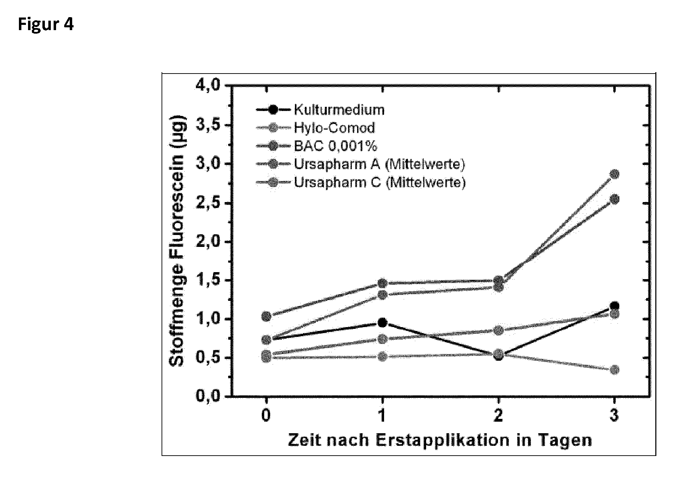

### Vergleich der Daten (Figur 4)

**Figur 4** stellt eine Zusammenstellung der oben aufgeführten Permeations-Daten dar. Für eine bessere Übersichtlichkeit sind für die dreifach bestimmten Permeations-Daten der Testsubstanzen Ursapharm A und C hier jeweils Mittelwerte der gemessenen Stoffmengen angegeben. Erkennbar ist, dass das mittels des erfindungsgemäßen Vehikelsystems (Ursapharm A) eine verbesserte Permeation von Fluorescein im Vergleich zu allen untersuchten Zusammensetzungen erzielt werden kann.

### III. Analyse der Epithel-Regeneration nach wiederholter Applikation von Ursapharm A (Lecithin-Gel + IPM 0,4 %)

Im Anschluss an die Hornhautkultur unter wiederholter Applikation von Ursapharm A (Lecithin-Gel + IPM 0,4 %, Experiment 4) wurde diese Hornhautkultur unter Standard-Kulturbedingungen für weitere 2 Tage kultiviert. Dazu 72 Stunden nach Erstapplikation der Testsubstanz Ursapharm A (Lecithin-Gel + IPM 0,4 %) die Kultur lediglich unter der stündlichen Simulation des Lidschlages mit Kulturmedium fortgeführt. Die Hornhaut wurde 48 Stunden nach letztmaliger Applikation von Ursapharm A (Lecithin-Gel + IPM 0,4 %) nochmals mittels OCT und Makroskopie untersucht. Ebenfalls wurde zu diesem Zeitpunkt ein weiterer Wert für die Fluorescein-Permeation bestimmt.

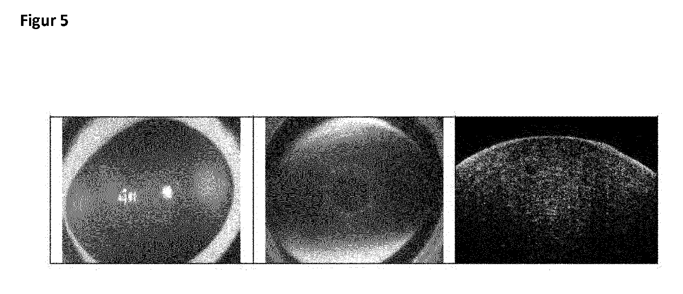

Ergebnis: Das Ergebnis dieses Experiments ist in **Figur 5** dargestellt. Von links nach rechts:
Makroskopie mit Auflicht, Makroskopie mit Durchlicht, optische Kohärenztomographie. Deutlich ist zu erkennen, dass das Epithel, ausgehend von den limbalen Stammzellen, innerhalb der 48 Stunden ohne Applikation von Ursapharm A (Lecithin-Gel + IPM 0,4 %) nahezu vollständig regeneriert ist.

### IV. Diskussion:

In den Langzeitversuchen, basierend auf Experimenten mit vitalen Hornhautkulturen im EVEIT - longterm System, wird wie erwartet eine hervorragenden Epithelintegrität bei wiederholter Applikation von HYLO-COMOD^{®} beobachtet. Dies bestätigt sich in den gemessenen Werten der Fluorescein-Permeation durch die mit HYLO-COMOD^{®} betropften Hornhäute. Die Applikation von HYLO-COMOD^{®} führt hier zu einer Konstant niedrigen Permeabilität über die gesamte Kulturzeit von 3 Tagen. Bei Standard-Kulturbedingungen, mit einer Simulation des Lidschlages mittels Applikation von Kulturmedium, wird während der Kulturzeit ein geringer Anstieg der Permeabilität beobachtet. Aus diesem Vergleich kann eine Schutzwirkung von HYLO-COMOD^{®} auf die *Zonula occludens* (Tight Junctions) des Hornhautepithels abgeleitet werden. Histologisch entsprechen sowohl die nach Standard-Kulturbedingungen kultivierte Hornhäute als auch die Hornhäute mit HYLO-COMOD^{®} Applikation morphologisch einer vollkommen intakten Hornhaut. Damit entsprechen diese beiden Kulturbedingungen beide dem erwarteten Verhalten einer Negativkontrolle. Ein ähnliches Verhalten wird für die Testsubstanz Ursapharm C beobachtet. Hornhäute, auf welche die Testsubstanz Ursapharm C wiederholt appliziert wurde, zeigen ebenfalls innerhalb der Beobachtungszeit von 3 Kulturtagen sowohl in der optischen Kohärenztomographie als auch makroskopisch keinerlei Auffälligkeiten. Die Substanz Ursapharm C erhält die Epithelien hervorragend und erzielt damit ein gleichwertiges Verhalten wie die Negativkontrollen Kulturmedium (Experiment 4) und HYLO-COMOD^{®} (Experiment 5). Auch bezogen auf die Hornhautdicke ist Substanz C den Negativkontrollen zumindest gleichwertig. Histologisch wurde in den zwei untersuchten Kulturen, welche mit Ursapharm C betropft wurden, eine Anzahl von 4-5 Flügelzellschichten beobachtet. Üblicherweise sind in der Kaninchenhornhaut lediglich 2, maximal 3 Zellschichten histologisch nachweisbar. Anders als bei der Negativkontrolle HYLO-COMOD^{®} wird bei Ursapharm C keine Schutzwirkung auf die Zonula occludens (Tight Junctions) beobachtet. Die Fluorescein-Permeation steigt hier, vergleichbar mit den Standard-Kulturbedingungen leicht mit der Kulturdauer an. Einen Verlust der Integrität der Epithelien erleiden die Hornhäute unter Betropfung mit der als Positivkontrolle verwendeten Lösung von Benzalkoniumchlorid (0.001 %) in Ringerlösung. Gleiches gilt für die Integrität des epithelialen Verbandes unter der Betropfung mit Ursapharm A (Lecithin-Gel + IPM 0,4 %). Der Verlust der epithelialen Integrität ist sowohl in der OCT aufgrund der abnehmenden Epithelschichtdicke und einer deutlich ansteigenden Hornhautschichtdicke als auch durch einen positiven Fluorescein-Nachweis erkennbar. Diese Beobachtung bestätigt sich sowohl im histologischen Befund als auch in der sehr deutlich ansteigenden Fluorescein-permeation durch die Hornhaut. Der Umfang der beobachteten Zunahme der Hornhautschichtdicke bestätigt den Befund einer epithelialen Störung und schließt gleichzeitig endotheliale Störungen aus, welche nach unseren bisherigen Erfahrungen mit Endothelschäden mit einer nochmals wesentlich höheren Schichtdickenzunahme einhergehen würden. Ein zusätzlicher Hinweis auf den Verlust der Epithelintegrität ist auch die Beobachtung, dass sich die epitheliale Schichtdicke am dritten Tag nach Erstapplikation von Ursapharm A (Lecithin-Gel + IPM 0,4 %) unter moderatem Verdunstungsstress (relative Luftfeuchte 30 % anstelle der Kulturbedingungen > 95 %) innerhalb von Minuten verringert. Ein solches Verhalten ist bei den Negativkontrollen nicht zu beobachten. Dies mag an der Detergenz-Wirkung von Lecithin liegen.

Nach Applikation der erfindungsgemäßen Zusammensetzung (Experiment 4) hat sich in Versuchen mit exzidierten Hornhäuten überraschend gezeigt, dass die Barrierefunktion der Hornhaut in einer reversiblen Art und Weise herabgesetzt wird. Beim Betropfen mit der erfindungsgemäßen Zusammensetzung wird die Integrität des Epithelverbandes herabgesetzt. Dies bewirkt eine effektive Wirkstoff-Permeation durch die Cornea. Dies wurde in den Versuchen anhand der Modellsubstanz Fluorescein belegt, die als Modell für überwiegend parazellulär resorbierte Wirkstoffe eingesetzt wird. Im Gegensatz zum Betropfen mit der Positivkontrolle HYLO-COMOD, wurde bei Behandlung mit der erfindungsmäßigen Zusammensetzung ein sehr deutlicher Anstieg der Fluorescein-Permeation durch die Hornhaut gemessen. Überraschend hat sich gezeigt, dass eine rasche "Nachheilung" und Proliferation der Epithelzellen erfolgt.

Die Applikation von Lecithin als biologisches Detergenz hat ebenso schwere Folgen wie Benzalkoniumchlorid. Im Gegensatz dazu behindert diese Substanz in der "Nachheilung" allerdings die Proliferation der Epithelien nicht. Dies mag als Vorteil eingesetzt werden in Fällen von Medikamenten, wo eine Permeationserhöhung erreicht werden soll, ohne den toxischen Schaden des Benzalkoniumchlorides zu verursachen. Dieses Ergebnis steht im krassen Gegensatz zur Behandlung mit Benzalkoniumchlorid (0,001%) (Experiment 3) in den Versuchen, bei dem zwar ebenso eine Permeation des Fluoreszins beobachtet werden konnte. Eine "Nachheilung" der Epithelzellen erfolgen in diesem Falle nicht. Die Zellen waren irreversibel geschädigt; gleichzeitig wird das Niveau der Fluorescein-Permeation mit den erfindungsgemäßen Zusammensetzungen nicht erreicht.

Dies erfindungsgemäßen Zusammensetzung weist damit einen klinisch sehr relevanter Vorteil auf: sie induziert eine vorübergehende Penetrationserhöhung zur verbesserten Wirkstoffaufnahme ins Augeninnere, ohne den toxischen Schaden des Benzalkoniumchlorids zu verursachen.

### V. Untersuchung des Brechungsindex der erfindungsgemäßen Zusammensetzung

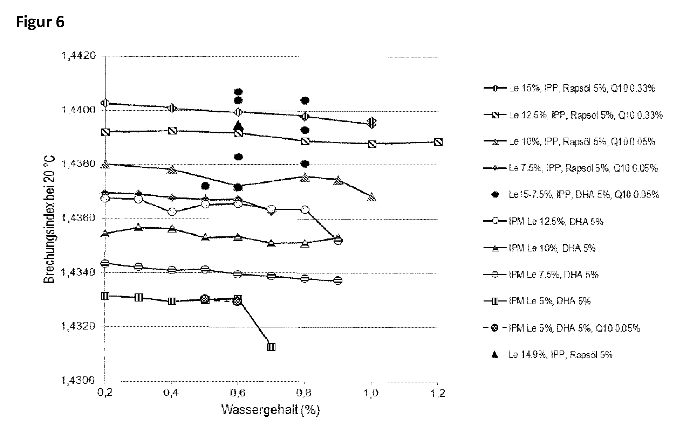

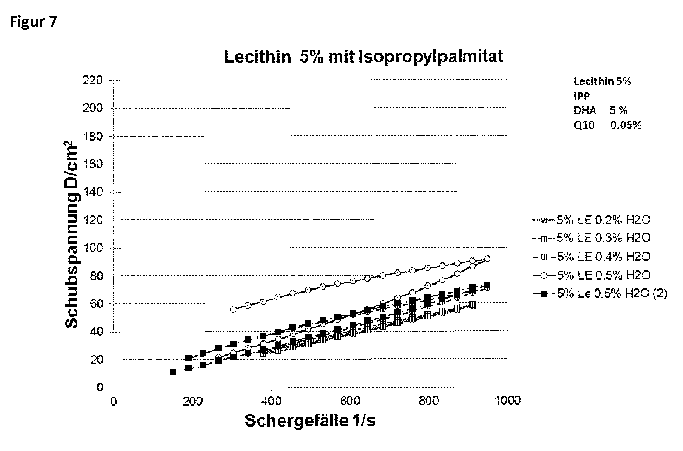

Untersuchungen der Brechungsindices (bei 35°C) beispielhafter Vehikelsysteme gemäß der Erfindung ergaben, dass diese innerhalb des für den ophthalmologischen Anwendungsbereich geeigneten Werte liegen. In **Figur 6** sind die Versuchsergebnisse abgebildet (Le = Lecithin, DHA = Docosahexaensäure). Alle Formulierungen weisen dabei Brechungsindices im Bereich zwischen 1,43 und 1,442 auf.

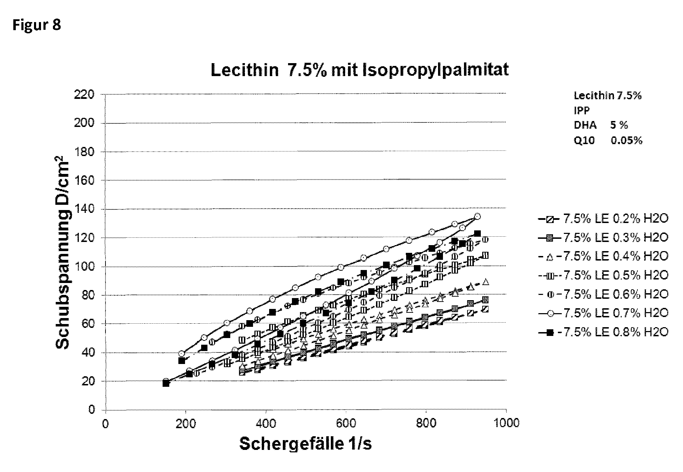

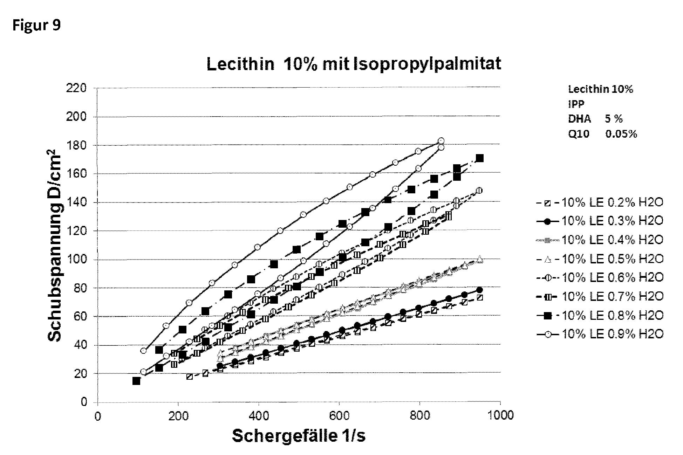

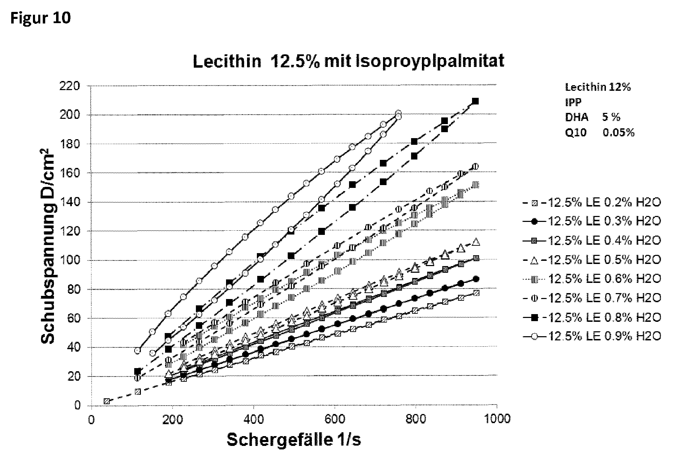

### VI. Rheometrische Untersuchungen verschiedener Vehikelsysteme (Figuren 8 bis 10)

Das Fließverhalten von ophthalmischen Formulierungen, vor allem von Gelen und Salben, spielt eine wichtige Rolle für das Empfinden bzw. das Fremdkörpergefühl der Formulierung im Auge.

Unter Thixotropie versteht man sowohl den Rückgang der Viskosität unter Scherung im Verlauf der Zeit als auch das Ansteigen der Viskosität im Verlauf der Zeit, wenn die Probe nicht mehr oder nur noch wenig geschert wird.

Die rheologischen Messungen (bei 35°C) belegen die thioxotrophen Eigenschaften beispielhafter Vehikelsysteme gemäß der Erfindung und daher zeigen deren Eignung für den ophthalmologischen Anwendungsbereich.

Die Abbildungen 8 bis 10 zeigen die Fließkurven für die untersuchten Formulierungen (Lecithin mit 7,5 Gew.-%, 10 Gew.-% bzw. 12,5 Gew.% Isopropylpalmitat). Die Fließkurven charakterisieren das Fließverhalten bei unterschiedlichen Scherraten. Scherkräfte/ Scherraten treten im Auge beim Lidschlag auf. Der Verlauf der Schubspannung τ ist in Abhängigkeit von der Scherrate dargestellt. Deutlich zu sehen ist die aufgespannte Hysteresefläche zwischen den Messkurven der über einen Bereich gesteigerten und dann wieder schrittweise verlangsamten Schergeschwindigkeit. Die Kurve kehrt, wie in der Abbildung zu sehen ist, nicht in sich selbst zurück. Unter Scherbelastung nimmt also mit der Zeit die Viskosität ab. Daher sind die Hin- und Rückkurve nicht identisch. Nach Aussetzung der Scherbeanspruchung wird die Ausgangsviskosität wieder aufgebaut. Vereinfacht heißt das, die thixotrope Flüssigkeit wird mit der Dauer ihrer Deformation immer dünnflüssiger. Nach Beendigung der Scherbelastung steigt die Viskosität zeitabhängig wieder an.

Diese Eigenschaft ist wünschenswert für Augenformulierungen, da beim Lidschlag die Viskosität der Formulierung abnimmt dadurch leichter vom Augenlid über die Augenoberfläche verteilt wird und nicht als Fremdkörper im Auge wahrgenommen wird.

### VII. Vitalitätsbestimmung an cornealen Epithelzellen in Kultur nach Applikation verschiedener Proben

Hintergrund der Untersuchung war es unterschiedliche Formulierungen, die eine hervorragende Gel-Stabilität und Konsistenz gezeigt hatten, auf Zytotoxizität testen zu lassen.

Der Zytotoxzititätstest wurden an der humanen cornealen epithelialen Zelllinie HCE-T durchgeführt, also im Zell-Test. Die Kultivierung erfolgte in einer schwarzen 96er Well Platte (Nunc). Pro Well wurden 25.000 Zellen ausgesät und für 48 h kultiviert. Die Bestimmung der Viabilität wurde mit dem Cell Titer Blue^{®} Test durchgeführt. Hierbei wird den Zellen nach der Exposition mit den Proben der Farbstoff Resazurin zugesetzt. Viable Zellen sind in der Lage, aufgrund ihrer mitochondrialen Aktivität das Resazurin in das fluoreszierende Resorufin umzusetzen, welches fluorimetrisch bestimmt werden kann (Abbildung 1). Über Positivkontrolle (Krebs-Ringer-Puffer -KRB und salinischer Phosphatpuffer - PBS) sowie Negativkontrolle (Lysispuffer) lässt sich die Viabilität der Zellen in Prozent ausdrücken. Alle Proben wurden mit n=6 bestimmt. Zur Bestimmung wurde das Zellkulturmedium entfernt, die Zellen mit KRB gespült und für 60 Minuten mit den verschiedenen Proben sowie Kontrollen inkubiert. Danach wurden die Flüssigkeiten entfernt und dreimal mit KRB plus 1% Polysorbat 80 gespült, um Reste der Proben zu entfernt. Danach wurden alle Proben mit KRB und Resazurin nach Protokoll von Promega behandelt und mit einem Genies Plattenreader (Tecan) vermessen.

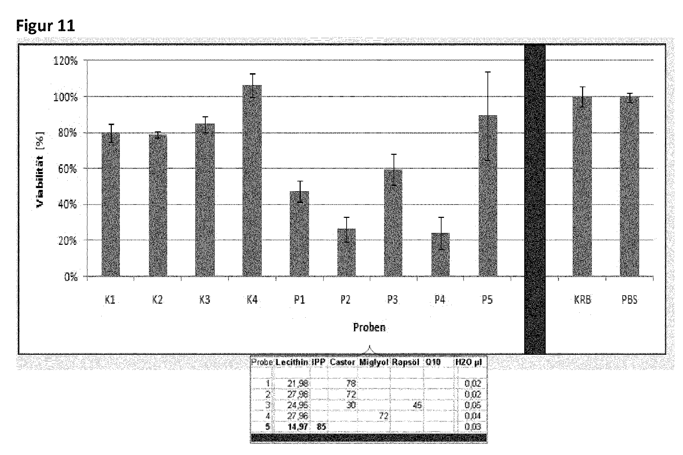

In Figur 11 sind Versuchsergebnisse Vitalitätsbestimmung an cornealen Epithelzellen in Kultur dargestellt. Es zeigte sich für die Proben K1-4 eine Viabilität von 80-100% und für die Proben P1-6 in der Regel eine deutlich reduzierte Viabilität im Bereich von 25-90%. Die Proben P2, 4 und 6 zeigten im Vergleich die niedrigsten Werte für die Viabilität. Die Proben P1 und 3 zeigten Werte um 50% und für die Probe P5 ließ sich e{n Wert um 90% detektieren, wenngleich für die letztgenannte Probe höhere Werte für die Standardabweichung bestimmt wurden.

### Vergleich der Kontrollen K1 bis K4:

Die verschiedenen Gelgrundlagen, also apolaren Phasen Castor-Öl (K1),

Miglyol (K2), Rapsöl (K3) und Isoprpylpalmitat (IPP, K4) wurden auf Zytotoxizität getestet. Die geringste Zyotoxozität im Zelltest und damit beste Verträglichkeit weist IPP auf. Nach Behandlung mit dieser Grundlage überleben 100% der Zellen, im Gegensatz zu einer Überlebensrate von ca. 80% nach Behandlung mit allen anderen getesteten Grundlagen.

### Vergleich der Proben:

Die Proben P1 bis P5 wurden in Bezug auf maximale Gel-Stabilität und Konsistenz optimiert. Folglich sind neben der apolaren Grundlage auch unterschiedliche Lecithin-Konzentrationen gewählt.

Der Vergleich von P1 und P2 zeigt, dass eine geringere Lecithin-Konzentration bei gleicher Grundlage (Castoröl), einen geringeren zytotoxischen Effekt ausübt. Die Lecithin-Konzentrationen, die ausgewählt wurden wegen einer hervorragenden Gel-Stabilität, zeigen im Zytotoxizitätstest eine zu schlechte Verträglichkeit. Die Lecithin-Konzentrationen müssen für eine gute Verträglichkeit noch weiter reduziert werden. Auch die Probe P3 bestätigt diesen Trend.

Die Probe P4 mit einer vergleichbaren Lecithin-Konzentration wie P2, aber der Grundlage Miglyol, zeigt eine ähnliche Zytotoxizität von ca. 75%.

Die Probe 5, die bereits auf der verträglichsten Grundlage IPP beruht und mit rund 15%, die geringste Konzentration an Lecithin von den getesteten Proben aufweist, zeigt die beste Verträglichkeit mit einer Überlebensrate von 100% der Zellen.

Diese Versuchsergebnisse im Zell-Test mit humanen, cornealen Epithelzellen, weisen darauf hin, dass eine Lecithin-Konzentration von kleiner/gleich 15 % vorteilhaft ist für die Verträglichkeit der Formulierung. Das Zellmodell stellt ein sehr sensitives Testsystem dar.

## Patentansprüche

1. Ophthalmologisches Vehikelsystem, enthaltend
a) ≥ 30 bis 99,95 Gew.-%, bezogen auf die gesamte Zusammensetzung mindestens einen Fettsäureester,
b) 0,05 Gew.-% bis ≤ 50 Gew.-%, bezogen auf die gesamte Zusammensetzung einen oder mindestens einen Emulgator
zur Permeation und/oder zum Wirkstofftransport von ophthalmologischen Wirkstoffen durch die Hornhaut (Cornea) und/oder die Bindehaut (Sklera) des Auges eines Säugetieres bei der Prophylaxe und/oder Behandlung von Erkrankungen des vorderen, und/oder hinteren Augenabschnittes.

2. Ophthalmologisches Vehikelsystem nach Anspruch 1, **dadurch gekennzeichnet, dass**
a) der Fettsäureester ausgewählt ist aus der Gruppe bestehend aus Isopropylmyristat und Isopropylpalmitat und/oder
b) Emulgator ausgewählt ist aus der Gruppe der Lecithine, bevorzugt Phosphatidylcholin-haltiger Zusammensetzungen mit einem Phosphatidylcholin-Gehalt von mindestens 90 Gew.-%, weiter bevorzugt mindestens 95 Gew.-%, insbesondere Epikuron 200 ist.

3. Ophthalmologisches Vehikelsystem nach einem der vorhergehenden Ansprüche, **dadurch gekennzeichnet, dass**
a) der Gesamtgehalt des mindestens einen Fettsäureesters, bezogen auf die gesamte Zusammensetzung von 50 bis 99,9 Gew.-%, bevorzugt 70 bis 99,9 Gew.-% und/oder
b) der Gesamtgehalt des einen oder mindestens einen Emulgators, bezogen auf die gesamte Zusammensetzung von 0,1 bis 15 Gew.-%, bevorzugt von 5 bis 12 Gew.-%, besonders bevorzugt von 7 bis 10 Gew.-%
beträgt.

4. Ophthalmologisches Vehikelsystem nach einem der vorhergehenden Ansprüche, enthaltend mindestens eine ω-3-Fettsäure und/oder ein ω-3-Fettsäurederivat ausgewählt aus der Gruppe bestehend aus Estern, Mono-, Di- oder Triglyceriden, Lipiden, Oxygenierungsprodukten, Carboxylatsalzen, Amiden, sonstigen pharmakologisch akzeptablen Carbonsäurederivaten und Mischungen hiervon.

5. Ophthalmologisches Vehikelsystem nach vorhergehendem Anspruch, **dadurch gekennzeichnet, dass** die mindestens eine ω-3-Fettsäure ausgewählt ist aus der Gruppe bestehend aus α-Linolensäure, Stearidonsäure, Eicosatetraensäure, Eicosapentaensäure (EPA), Docosapentaensäure (DPA), Docosahexaensäure, Hexadecatrienoensäure, Eicosatrienoensäure, Heneicosapentaenoensäure, Tetracosapentaenoensäure, Tetracosahexaenoensäure, hiervon abgeleitete Oxygenierungsprodukte sowie Mischungen oder Kombinationen hieraus.

6. Ophthalmologisches Vehikelsystem nach einem der beiden vorhergehenden Ansprüche, **dadurch gekennzeichnet, dass** die mindestens eine ω-3-Fettsäure in Form
a) eines Esters eines organischen Alkohols, bevorzugt eines linearen oder verzweigten aliphatischen einwertigen Alkohols mit 1 bis 18 Kohlenstoffatomen, besonders bevorzugt als Methyl-, Ethyl-, n-Propyl-, i-Propyl-, n-Butyl-, i-Butyl-, t-Butylester enthalten ist und/oder
b) eines pflanzlichen oder tierischen Öls enthalten ist, das neben der mindestens einen ω-3-Fettsäure noch mindestens eine ω-6-Fettsäure enthält, wobei das molare Verhältnis der ω-3-Fettsäure zur ω-6-Fettsäure von 100:1 bis 1:100, bevorzugt 20:1 bis 1:10, weiter bevorzugt 15:1 bis 1:1, besonders bevorzugt von 8:1 bis 2:1 beträgt,
vorliegt.

7. Ophthalmologisches Vehikelsystem nach einem der Ansprüche 4 bis 6, **dadurch gekennzeichnet, dass** die mindestens eine ω-3-Fettsäure in Form eines Öls, ausgewählt aus der Gruppe bestehend aus Algenöl, Fischöl, Perillaöl, Shiöl, Leinöl, Leindotteröl, Sacha Inchi Öl, Rapsöl, Olivenöl, Nachtkerzenöl, Sojaöl, Hanföl, Walnussöl, Erdnussöl, Sesamöl, Maiskeimöl, Flachssamenöl und/oder Mischungen hieraus vorliegt.

8. Ophthalmologisches Vehikelsystem nach einem der Ansprüche 4 bis 7, **dadurch gekennzeichnet, dass** der Gehalt der mindestens einen ω-3-Fettsäure und/oder des Derivats hiervon, bezogen auf die gesamte Zusammensetzung, zwischen 0,01 und 60 Gew.-%, bevorzugt zwischen 0,05 und 30 Gew.-%, besonders bevorzugt zwischen 0,1 und 10 Gew.-% beträgt.

9. Ophthalmologisches Vehikelsystem nach einem der vorhergehenden Ansprüche, **dadurch gekennzeichnet, dass** die Zusammensetzung frei ist von Verbindungen, ausgewählt aus der Gruppe bestehend aus quartären Ammoniumverbindungen, wie Benzalkoniumchlorid; DMSO, Natriumglycocholat und/oder Natriumfusidat.

10. Ophthalmologisches Vehikelsystem nach einem der vorhergehenden Ansprüche, enthaltend mindestens einen ophthalmologischen Wirkstoff in einer pharmazeutisch wirksamen Konzentration, insbesondere mindestens einen Wirkstoff ausgewählt aus der Gruppe bestehend aus Antibiotika, Corticoide, Lokalanästhetika, abschwellenden Medikamenten, nichtsteroidalen Antiphlogistika, Virustatica, Antiseptika, Kortison, antiallergischen Wirkstoffen, Prostaglandin-Analoga, Wirkstoffen aus der Wirkstoffklasse der Antihistaminika und/oder der Corticosteroide, antiallergischen Wirkstoffe, Pantothensäurederivaten, nichtsteroidalen Entzündungshemmer, Vaskokonstriktoren und/oder Anti-Glaucom-Wirkstoffen.

11. Ophthalmologisches Vehikelsystem nach einem der vorhergehenden Ansprüche, enthaltend mindestens einen weiteren Bestandteil, ausgewählt aus der Gruppe bestehend aus
a) mindestens einen anti-inflammatorisch und/oder antioxidativ und/oder antiallergisch wirkenden Stoff, ausgewählt aus der Gruppe bestehend aus Flavonoiden (z.B. Rutin, Quercetin, Curcurmin), Isoflavonoiden (z.B. Silymarin), Polyphenole (z.B. Resveratol), Anthocyanen, Triterpenen, Monoterpenalkoholen, Phenolcarbonsäuren, Carotinoiden (z.B. β-Carotin, α-Carotin, Lycopin, β-Cryptoxanthin, Lutein, Zeaxanthin), Retinoide (z.B. Tretinoin), Tocopherolen (Vitamin E) und Biotin, Vitamine A, C, D, K, Coenzym Q (=Q10) Carnitin, N-Acetyl-Carnitin, Glutathion, Carnesol, Ubichinon und/oder Taurin und/oder pflanzliche Einzelstoffe, Stoffgemische, ein flüssiger oder fester Extrakt, ein Destillat oder ein Öl oder ätherisches Öl, bevorzugt aus Pflanzen der Gattungen oder Arten Rosmarin, Sanddorn, Myrrhe, Eupharis (Augentrost), Kamille, Arnika, Ringelblume, Thymian, Echina, Calendula, Teebaum, Teestrauch, Apfelbeere (Aronia), Ginkgo, Ginseng, Heidelbeere, Holunder, Lavendel, Anis, bevorzugt in einer Menge von 0,01 und 5 Gew.-%, bezogen auf de gesamte Zusammensetzung,
b) mindestens einen Gelbildner, ausgewählt aus der Gruppe bestehend aus natürlichen oder synthetischen Polymeren, bevorzugt in einer Menge zwischen 0,01 und 5 Gew.-%, bezogen auf die gesamte Zusammensetzung,
c) mindestens ein Verdickungsmittel, bevorzugt in einer Menge zwischen 0,5 und 5 Gew.-%, bezogen auf die gesamte Zusammensetzung.
d) mindestens ein Feuchthaltemittel,
e) mindestens eine Hilfsstoff, ausgewählt aus der Gruppe bestehend aus anorganischen Puffersubstanzen, organischen Puffersubstanzen, anorganischen Salzen, organischen Salzen, Viskositätsreglern, Lösungsmitteln, Lösungsvermittlern, Lösungsbeschleunigern, Salzbildnern, Viskositäts- und Konsistenzbeeinflussern, Solubilisatoren, Benetzern, Spreizmitteln, Füll- und Trägerstoffen, Osmolaritätsreglern sowie Mischungen davon, sowie
f) Kombinationen aus den zuvor genannten Bestandteilen.

12. Ophthalmologisches Vehikelsystem nach einem der vorhergehenden Ansprüche in flüssiger, viskoser oder halbfester Form, insbesondere in Form eines Gels, eines thixotropen Gels, eines Lipogels, eines Organogels, eines Mikroemulsionsgels, eines Sprühgels, einer Wasser-in-Öl-Emulsion, oder eines in situ Gels.

13. Ophthalmologisches Vehikelsystem nach einem der vorhergehenden Ansprüche zur Prophylaxe und/oder Behandlung von Entzündung (z.B. Uveitis, Iritis, Chorioiditis, Azoor (Acute zonal occult outer retinopathy/Akute zonale okkulte äußere Retinopathie), Neuritis nervi optici), Katarakt (Grauer Star), Glaucom, Retinopathie, Makuladegenerationen (AMD), Netzhautablösung, Retinoblastom und/oder Aderhautmelanom und/oder zur Vor- und/oder Nachbehandlung chirurgischer Eingriffe am Auge, insbesondere chirurgischer Eingriffe ausgewählt aus der Gruppe bestehend aus chirurgischen Eingriffen am vorderen Augenabschnitt, Kataraktextraktion mit Linsenimplantation, refraktiv-chirurgischen Eingriffen, Eingriffen an der Hornhaut und Hornhauttransplantationen und/oder Eingriffen an der Bindehaut.

14. Ophthalmologisches Vehikelsystem nach einem der vorhergehenden Ansprüche zur topischen Applikation am Auge, insbesondere durch Einträufeln oder Eintropfen ins Auge bzw. auf die Augenoberfläche, Einsprühen oder Aufsprühen ins Auge bzw. auf die Augenoberfläche, durch Einträufeln oder Eintropfen oder als Geldepot in den/ im Bindehautsack oder als Insert.

15. Ophthalmologisches Vehikelsystem nach vorhergehendem Anspruch, **dadurch gekennzeichnet, dass** die Applikation 1 mal täglich bis 1 mal stündlich, bevorzugt 1 mal täglich bis 4 mal täglich erfolgt.

16. Ophthalmologisches Kit, umfassend
a) ein Ophthalmologisches Vehikelsystem nach einem der vorhergehenden Ansprüche sowie
b) eine ophthalmologische Wirkstoffformulierung als separate Formulierungen.

17. Verwendung einer Zusammensetzung, enthaltend
a) ≥ 30 bis 99,95 Gew.-%, bezogen auf die gesamte Zusammensetzung mindestens einen Fettsäureester,
b) 0,05 Gew.-% bis ≤ 50 Gew.-%, bezogen auf die gesamte Zusammensetzung einen oder mindestens einen Emulgator
als Vehikelystem, Penetrationsbeschleuniger, Penetrationsenhancer, Absorptionsenhancer/-verbesserer/-beschleuiger zur Permeation und/oder zum Wirkstofftranport von ophthalmologischen Wirkstoffen durch die Hornhaut (Cornea) und/oder die Bindehaut (Sklera) des Auges eines Säugetieres.

18. Fluidspender für ein keimfreies Fluid, mit
a) einem Durchgang, der eine Einlassöffnung für ein in einem Vorratsbehälter aus einem flexiblen Material enthaltenes Fluid und eine Ausströmöffnung zum Ausgeben des Fluids verbindet und darin wenigstens eine oligodynamisch aktive Substanz besitzt, die mit dem Fluid in Kontakt ist;
b) einer Dosierpumpe, die ohne Luftdruckkompensation arbeitet, umfassend ein Einlassventil zum Schließen der Einlassöffnung, wobei das Einlassventil ein Material aufweist, das mit dem Fluid über eine oligodynamisch aktive Substanz wechselwirken kann; und
c) eine Federeinrichtung, die in Kontakt mit dem Fluid steht, wobei das Einlassventil und die Federeinrichtung ein rostfreies Stahlmaterial als eine oligodynamisch aktive Substanz aufweisen und eine Dekontaminierungseinrichtung im oberen Teil des Auslasskanals vorgesehen ist, wobei die Dekontaminierungseinrichtung ein Material aufweist, das mit dem Fluid über eine oligodynamische Substanz wechselwirken kann, die ausgewählt ist aus der Gruppe bestehend aus Silber, Silbersalzen, anderen Silberverbindungen, Legierungen und Nanomeren davon in entweder metallischer oder Salzform oder als eine chemische Verbindung daraus,
**dadurch gekennzeichnet, dass**
das im Vorratsbehälter enthaltene Fluid ein ophthalmologisches Vehikelsystem nach einem der Ansprüche 1 bis 15 ist.
